Europäisches Patentamt

European Patent Office

Office européen des brevets

(19)

(11) Publication number: **0 336 966 B1**

(12) **EUROPEAN PATENT SPECIFICATION**

(45) Date of publication of patent specification: **13.07.94**   (51) Int. Cl.⁵: **C12N 5/00**, C12M 3/00, //C12M1/12

(21) Application number: **88907809.3**

(22) Date of filing: **05.09.88**

(86) International application number: **PCT/JP88/00890**

(87) International publication number: **WO 89/02458 (23.03.89 89/07)**

(54) **CELL CULTURE METHOD AND APPARATUS.**

(30) Priority: **07.09.87 JP 221914/87**
**06.05.88 JP 109028/88**

(43) Date of publication of application:
**18.10.89 Bulletin 89/42**

(45) Publication of the grant of the patent:
**13.07.94 Bulletin 94/28**

(84) Designated Contracting States:
**CH DE FR GB LI SE**

(56) References cited:
**EP-A- 0 073 079**
**EP-A- 0 270 908**
**JP-A- 6 188 872**
**JP-A-61 146 308**
**JP-A-61 283 311**

**PATENT ABSTRACTS OF JAPAN, vol. 10, no. 262 (C-371)(2318), 06 September 1986&NUM;**

**PATENT ABSTRACTS OF JAPAN; p. 25 C 422 (JP A 6128331)&NUM;**

(73) Proprietor: **HITACHI, LTD.**
**6, Kanda Surugadai 4-chome**
**Chiyoda-ku, Tokyo 101(JP)**

(72) Inventor: **ISHIDA, Masahiko**
**20-8, Hanayama-cho 1-chome**
**Hitachi-shi Ibaraki 317(JP)**
Inventor: **NISHIMURA, Yuusaku**
**1-6, Kanesawa-cho 6-chome**
**Hitachi-shi Ibaraki 316(JP)**
Inventor: **YAMAGUCHI, Tetsuo**
**43-26, Nishinarusawa-cho 4-chome**
**Hitachi-shi Ibaraki 316(JP)**
Inventor: **ITOH, Kazuyuki**
**30 Ridge Road**
**Dobbs Ferry New York, NY 10522(US)**
Inventor: **MATSUZAKI, Harumi**
**18-5, Daihara-cho 2-chome**
**Hitachi-shi Ibaraki 317(JP)**
Inventor: **BABA, Kenji**
**8-6, Mikanohara-cho 1-chome**
**Hitachi-shi Ibaraki 317(JP)**

Inventor: **SAITOU, Setsuo**
**26-3, Higashiohnuma-cho 2-chome**
**Hitachi-shi Ibaraki 316(JP)**
Inventor: **YAHAGI, Hayao**
**6-25, Nishinarusawa-cho 4-chome**
**Hitachi-shi Ibaraki 316(JP)**
Inventor: **KUBOTA, Masayoshi**
**2453-11, Zuiryu-cho**
**Hitachiohta-shi Ibaraki 313(JP)**
Inventor: **HAGA, Ryoichi**
**11-3, Daihara-cho 3-chome**
**Hitachi-shi Ibaraki 317(JP)**


(74) Representative: **Strehl Schübel-Hopf Groening**
**& Partner**
**Maximilianstrasse 54**
**D-80538 München (DE)**

## Description

The present invention relates to a method of culturing biological cells and particularly to a large-scale and high-concentration cell culture method and an apparatus therefor which enable an efficient removal of bubbles formed through submerged aeration.

In recent years, pharmaceuticals, such as interferon, have begun to be produced through not only culture of microbial cells, i.e., bacterial cells, commonly conducted in the art but also culture of animal cells.

In order to produce these pharmaceuticals on a commercial scale, it is necessary to use a culture method which enables cells to be cultured in a high concentration on a large scale in a stable state for a long period of time. For this reason, it is desired to develop a cell culture method satisfying the requirements for:

(1) a technique for separating biological cells from a medium and supplying a fresh medium; and

(2) a technique for supplying oxygen necessary for the growth of biological cells.

In order to satisfy the above-described requirement (1) , it is necessary to aseptically separate and concentrate cells suspended in a medium. When cells are cultured without replacement of the medium, the maximum cell concentration is about $2 \times 10^6$/ml in most cell systems. On the other hand, the cell concentration can be increased to at least $10^7$/ml by separating and concentrating the cells contained in the medium and continuing the culture in a fresh medium, which contributes to a reduction in the size of a culture tank and shortening of the culture time.

Although centrifugation is generally employed in a small-size test, it is difficult to aseptically conduct the centrifugation on a large scale from the viewpoint of the time taken for replacing the medium and complexness of the process. For this reason, a proposal has been made on a method wherein supply of a fresh medium to a culture tank and removal of a waste component produced as by-product from cells are conducted simultaneously with the separation and concentration of the cells. For example, Japanese Patent Publication No. 16635/1980 and Japanese Patent Laid-Open No. 265/1987 each propose a method wherein a cell sedimentation zone is provided within a culture tank and a fresh medium is supplied to the culture tank while discharging the supernatant of the sedimentation zone. Japanese Patent Laid-Open No. 257181/1986, Japanese Patent Publication No. 12989/1987, and Japanese Patent Publication No. 12990/1987 each propose a method wherein a hollow fiber membrane having a wall which permits permeation of a medium but does not permit permeation of cells is provided and the supply of a fresh medium and the removal of the by-products are conducted therethrough.

With respect to the above-described requirement (2), i.e., a technique for supplying oxygen, a means for directly passing air or oxygen-rich air through a medium has been adopted in the conventional culture of microorganisms etc. However, many of the liquid media used for culturing the cells or the cell-containing media are apt to cause bubbling. In particular, culture of animal cells wherein a serum is added brings about remarkable bubbling of the medium due to biopolymers contained in the serum and those secreted during culturing. The formed bubbles hardly break even when a shearing force is mechanically applied thereto, fill a gas phase portion of the culture tank, and finally overflow outside the system.

When the readily bubbling media are used, it is necessary to feed oxygen to the medium by dissolving oxygen in the medium so as not to bring about bubbling or breaking the bubbles formed through submerged aeration.

The former method has been mainly adopted in the art and comprises supplying oxygen to the gas phase portion of the culture tank to dissolve oxygen in the medium from the liquid surface. In this method, it it a common practice to agitate the medium for attaining improved contact of the medium with oxygen or to increase the amount of feed of oxygen for improving the contact of the oxygen with the liquid surface.

The above-described method is described in Japanese Patent Laid-Open Nos. 74574/1986, 36915/1986, and 259179/1985.

No effective debubbling technique has been developed with respect to a method of supplying oxygen through submerged aeration. Specifically, only one proposal has been made on a method wherein bubbles are mechanically broken with high-speed rotary blades in a culture method intended for some microorganisms having a strong cell wall and exhibiting a high proliferation rate (see Japanese Patent Laid-Open No. 13464/1979 and Japanese Patent Publication No. 12108/1981) . In this method, a high-speed drive portion should be provided within the tank, which makes the tank structure complicated. This method cannot be applied to animal cells because they are very weak against this kind of shearing force.

Further, JP-61-283 311 describes a debubbling system comprising a Teflon powder put on a mesh. However the system is used for debubbling in the culturing of microorganisms such as yeasts rather than the culturing of living cells. Strong bubbles as are formed in the process of culturing living cells cannot be effectively broken by the proposed solid material.

It is the object of the present invention to provide a cell culture method and an apparatus therefor which enables a medium be debubbled without detriment to proliferation of cells.

The above-described object can be attained by the method and apparatus as set out in claims 1 and 2.

The method of culturing cells according to the present invention is used for proliferation culture of biological cells and comprises a culture vessel, means for separating cells from a medium, means for removing a waste product, means for returning a medium after removal of the waste component to the culture container, and pipes for connecting these members.

In the culture vessel, i.e., a culture tank, biological cells are suspended in the medium. The medium contains an inorganic salt, glucose, an amino acid, an antibiotic substance, etc. and, if necessary, a serum.

The culture tank used for the culture method according to the present invention is characterized by comprising an aeration culture tank composed of a vessel for accommodating a medium, means for agitating the medium, means for separating cells from the medium, means for supplying the medium with a gas necessary for culture from near the bottom of the vessel, water-repellent debubbling means provided above the surface of the medium and having a number of gas-permeable openings, and means for detecting the progression of culturing.

In the present invention, although bubbling occurs when a gas is passed though a medium, the provision of porous debubbling means comprising a water-repellent material on the surface of the medium enables the bubbles to be broken. It is preferred that the water-repellent material for the debubbling means have a contact angle of at least 30° relative to a droplet of the medium. At least the surface layer of the above-described debubbling means comprises the above-described water-repellent material.

Brief Description of the Drawings

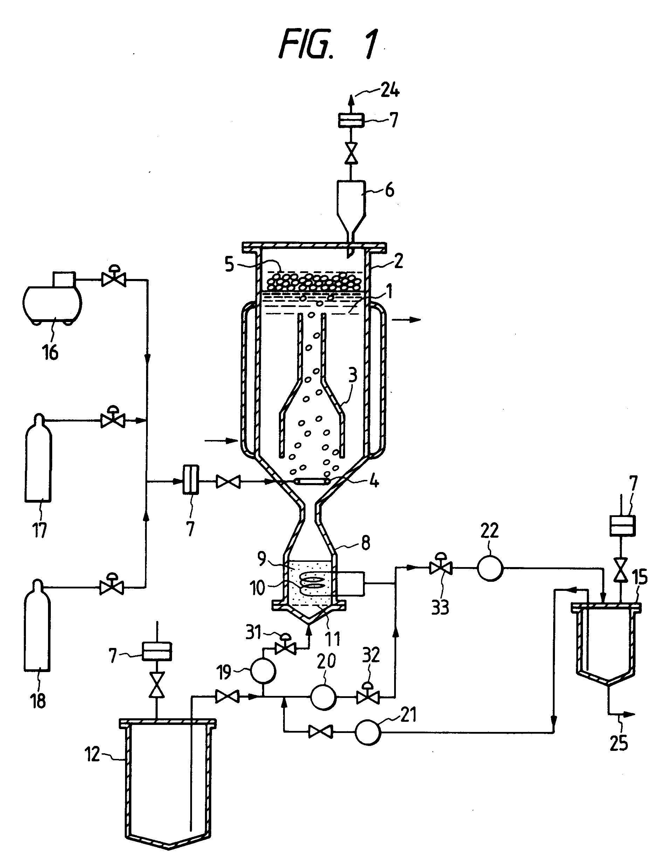

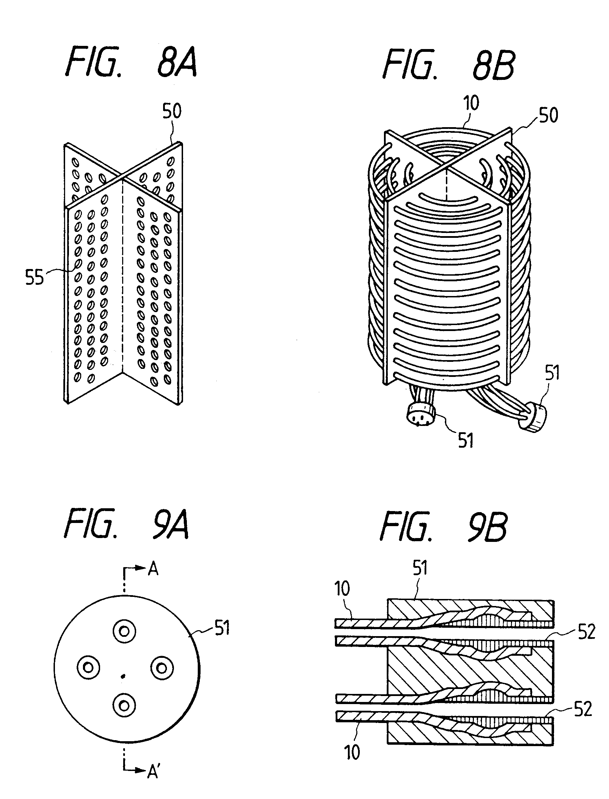

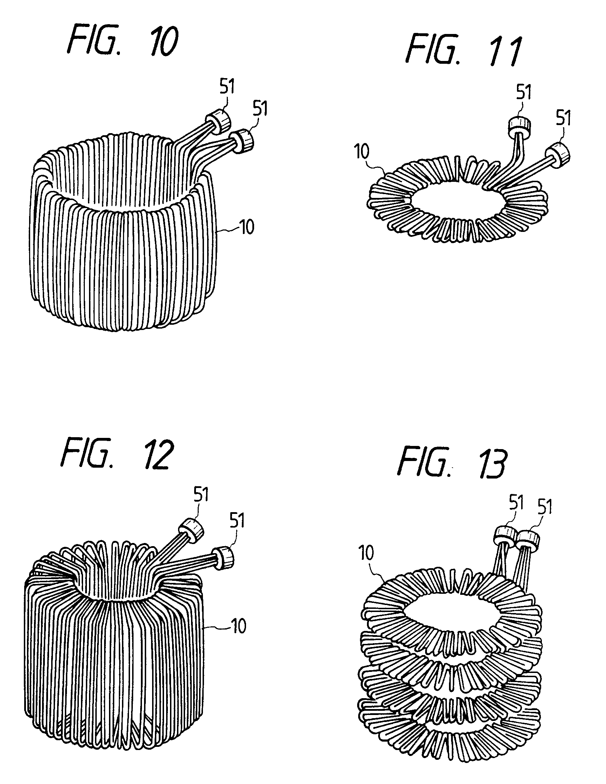

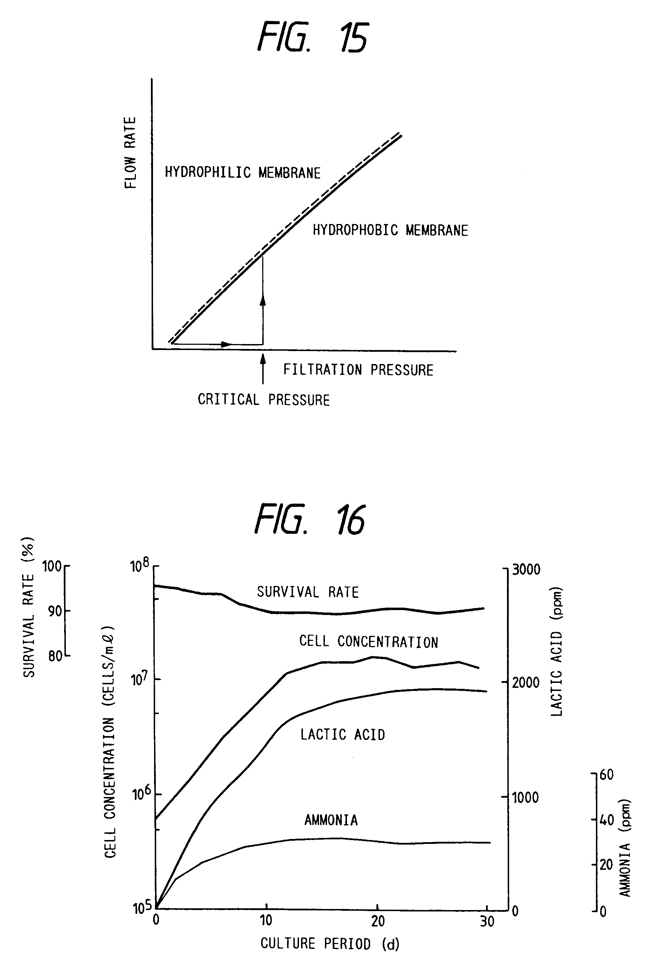

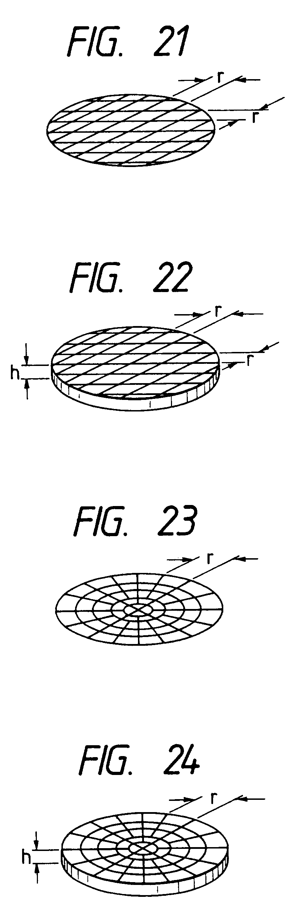

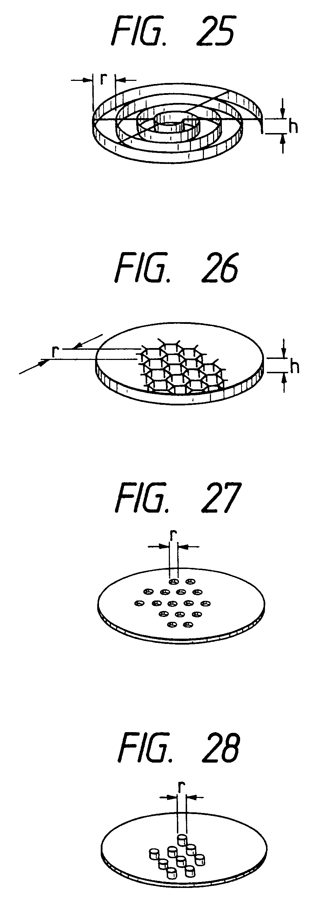

Figs. 1 to 7 and 14 are each a schematic view of an example of the present invention; Fig. 8A is a perspective view of a membrane supporting member; Fig. 8B is a perspective view of a filtration unit comprising the membrane supporting member shown in Fig. 8A and a hollow fiber membrane mounted thereon; Fig. 9 is a plan view and a cross-sectional view of a membrane supporting member; Figs. 10 to 13 are perspective views of shapes of filtration units; Fig. 15 is a graph showing the relationship between the filtration pressure of a hollow fiber membrane and the filtration flow rate; Figs. 16 to 20 are each a graph showing the relationship between the number of days for culturing cells and the cell concentration according to the present invention; and Figs. 21 to 28 are perspective views of debubbling layers.

Fig. 15 shows the permeability characteristics of hydrophilic and hydrophobic membranes. In the drawing, the broken line represents a graph with respect to the use of a hydrophilic membrane while the solid line represents a graph with respect to the use of a hydrophobic membrane.

The permeability characteristics of the hydrophilic membrane are proportional to the filtration pressure. On the other hand, in the case of the hydrophobic membrane, when a gas is present in the pores, i.e., before operation, no medium permeates through the membrane under a filtration pressure below a critical pressure (depending upon the hydrophobicity, pore diameter, membrane thickness, porosity in the inside of the membrane, etc.) until the pressure reaches the critical pressure. Once the medium permeates through the membrane, i.e., the pores of the membrane are filled with the medium, the permeability characteristics become equal to those of the hydrophilic membrane regardless of the filtration pressure. The permeability changes along an arrow on the solid line as shown in the drawing.

The critical pressure of a polytetrafluoroethylene hollow fiber (an outer diameter of 2 mm; an inner diameter of 1 mm) having a pore diameter of 0.8 $\mu$m is 1.5 kg/cm$^2$, and the permeability thereof after the pores are filled with the medium is about 0,75 ml/Pa•m$^2$•h (100 ml/mmHg•m$^2$•hr). This permeability is substantially equal to that of the hydrophilic membrane.

Therefore, when the filtration and backwashing are conducted by making use of the above-described hollow fiber, it is necessary to conduct actual operation prior to the first use of the hollow fiber membrane under a pressure above the above-described critical pressure to fill the pores of the membrane with the medium.

There is no particular limitation with respect to the hydrophobic hollow fiber membrane. However, it is necessary to use a hydrophobic hollow fiber membrane made of a hydrophobic material which has a sufficient strength and brings about no change in the pores for filtration even when exposed to an atmosphere of about 130°C. In this respect, a hydrophobic hollow membrane made of a tetrafluoroethylene resin is particularly preferred.

Although the hydrophobic filter membrane may be in the form of a long single hollow fiber membrane, it is preferred from the viewpoint of effectively utilizing the surface of the membrane that the membrane comprise a plurality of hollow fiber membranes in a bundled form.

When use is made of a hollow fiber membrane made of a tetrafluoroethylene resin which causes difficulty in the connection thereof through welding or bonding, an excellent membrane unit can be prepared by the following connecting method.

As shown in Fig. 9, a membrane expansion member 52 is inserted into both ends of the hollow fiber membrane 10. The membrane expansion member 52 is a hollow tube having an outer diameter larger than the inner diameter of the hollow fiber membrane, has such a shape that the cross-sectional area is gradually reduced towards the tip portion for the purpose of easily inserting the member into the hollow fiber membrane 10, and has a protruding portion having a large outer diameter on part of the surface thereof. The end of the hollow fiber membrane having the membrane expansion member 52 inserted thereinto is enclosed with a room-temperature curable resin varnish, and the resin varnish is cured. If necessary, the external shape is modified to prepare a membrane connecting member 51 used as an end connection to a filtration pipe. A filtration unit comprising a plurality of membranes can be prepared by bundling the ends of a plurality of hollow fiber membranes 10 and mounting the bundle on the same membrane connecting member 51. Examples of the hollow fiber membrane filtration unit are shown in Figs. 8 and 10 to 13.

The use of a hydrophobic membrane contributes to a remarkable reduction in the amount of cells or stains deposited on the surface of the membrane, which enables the membrane to be used for filtration for a long period of time.

After a predetermined amount of the medium is withdrawn, a fresh medium is reversely fed under pressure into the hydrophobic filter membrane for removal of cells and stains deposited on the surface of the filtration membrane. The backwash liquid may be a fresh medium, a liquid prepared by removing a waste component from a spent medium, or a mixture of them.

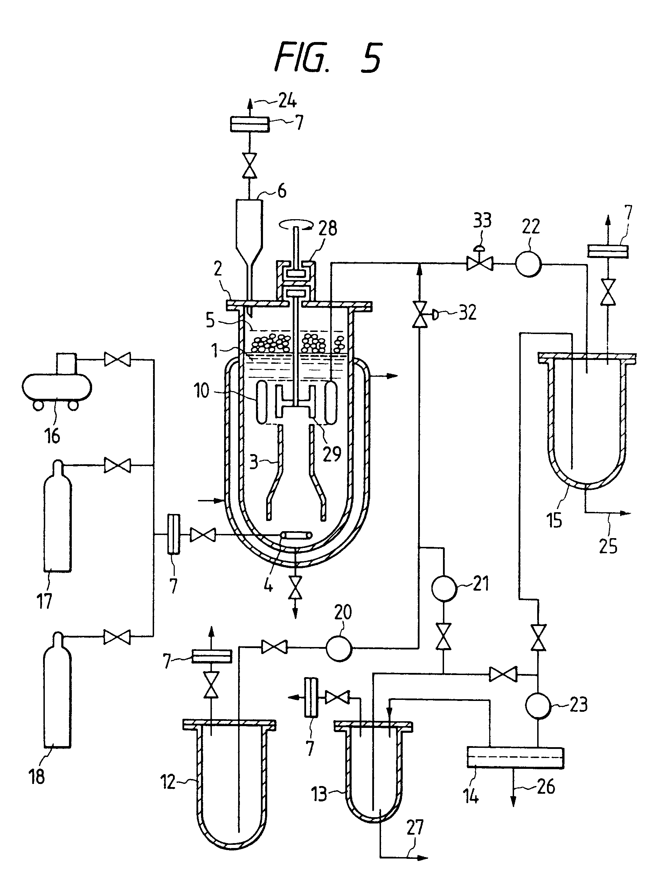

When the hydrophobic filter membrane 10 is provided in the medium contained in the culture tank 2 (Fig. 5), consideration should be given to the form of the membrane and location of provision thereof capable of maintaining smooth flow of the medium 1 and causing no deposition of cells derived from the occurrence of residence portion in the culture tank 2. In this case, it is desired to withdraw the medium at the lowest possible flow rate.

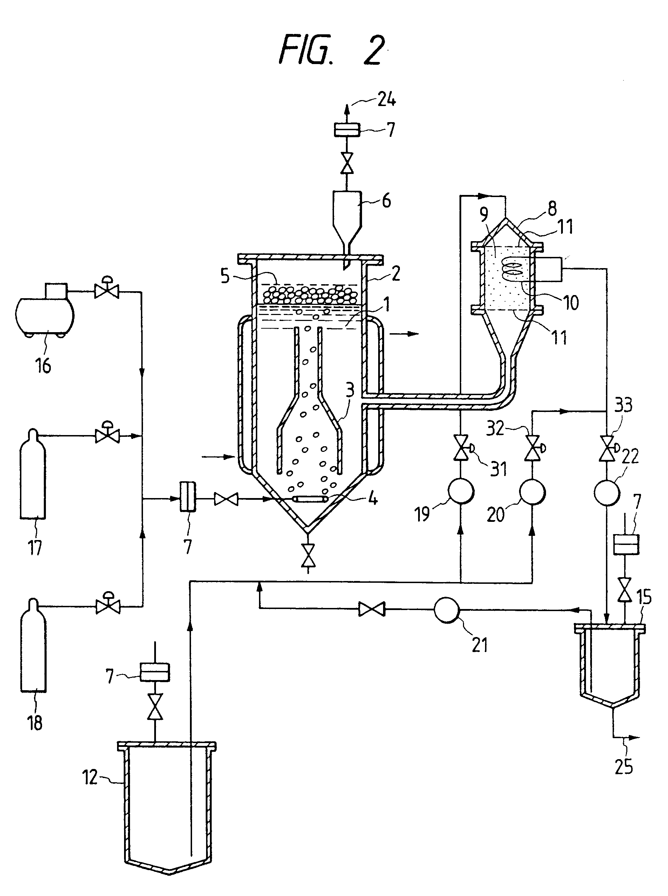

When the hydrophobic filter membrane 10 is provided outside the culture tank 2 (Fig. 2), it is housed in a filter unit 8. The filter unit 8 is connected to the culture tank 2 through a pipe or the like. When the hydrophobic filter membrane 10 is provided outside the culture tank 2, it is necessary to operate the apparatus in such a manner that the medium is withdrawn at the highest possible flow rate and the cells are returned to the culture tank as soon as the amount of withdrawal of the medium has reached a predetermined amount.

When the hydrophobic filter membrane 10 is housed in a filter unit 8 provided outside the culture tank 2, it is preferred that the membrane 10 used be a precoated membrane having a hydrophilic filter aid on the surface thereof. In this case, a filter 11 for supporting a filter aid 9 is provided within the filter unit 8 at the bottom or both the bottom and the top thereof near the hydrophobic filter membrane 10.

The filter 11 comprises a porous material, such as organic polymer material, glass, ceramic, or sintered metal, and a pore size thereof is selected so as to permit little or no permeation of particles of the filter aid 9 therethrough.

When a culture solution is introduced from the culture tank 2 into the filter unit 8, the cells contained in the medium are captured by the particles of the filter aid 9. The filter aid 9 is an organic polymer or an inorganic compound nontoxic to the cells, and it may be used alone or in combination of two or more of them. The material, particle diameter, shape, and the amount of packing thereof are determined depending upon the adhesion to cells, the particle diameter of cells, etc.

For example, with respect to cells having a particle diameter of 5 to 50 $\mu$m, when the cells are captured only by the so-called "screening effect" between particles of the filter aid 9, the particle diameter of the filter aid is substantially the same as that of the cells. On the other hand, when the cells adhere to the filter aid 9, it is preferred that the particle diameter of the filter aid 9 be several tens to several hundreds of $\mu$m. When a microcarrier bead is used, it is possible to conduct adherent culture. In the case of most cells, the filter aid 9 is selected through consideration of both of the above-described effects. Therefore, when the medium introduced into the filter unit 8 is passed through the filter aid 9 and reaches the hydrophobic filter membrane 10, the medium contains little or no cell, which enables the clogging of the hydrophobic filter membrane 10 to be substantially prevented even in the case of a long-term operation.

When the medium is fed into the filter unit 8 at a flow rate of about 0.1 to 10 $m^3/m^2 \cdot hr$ per unit surface area based on a characteristic inner diameter of the filter unit, it is possible to prevent the cells from being damaged by a shearing force caused when the medium is passed through between the particles of the filter aid 9. However, the flow rate varies depending upon the kind of cells, the particle diameter of the filter aid 9, the structure of the filter unit 8, etc.

By-products contained in the medium can permeate through the hydrophobic filter membrane 10. Therefore, in the above method, the cells contained in the medium are separated and concentrated, and the by-products such as waste products are discharged.

After a predetermined amount of the medium is discharged, a fresh medium is introduced into the filter unit 8. The term "fresh medium" used herein is intended to mean not only an unused medium but also a liquid prepared by removing the by-products of the cells contained in a spent medium. The composition of the medium may be different from that of the medium contained in the culture tank. When the above-described fresh medium is reversely introduced into the filter unit 8, the particles of the filter aid 9 ascend (or descend) and develop together with the cells captured in the above-described step.

In order to efficiently conduct the above-described step, the material, particle diameter, shape, etc. of the particles of the above-described filter aid 9 should be such that the filter aid particles settle (or are suspended in the culture solution) more rapidly than the cells. When a group of particles which settle more rapidly than the cells are used as the filter aid 9, it is desired that the fresh medium be fed into the filter unit 8 at such a flow rate that in the filter aid 9 to be developed, a group of particles exhibiting the highest sedimentation velocity slightly ascend. At that time, the filter aid 9 to be developed is substantially entirely developed, so that the cells are separated from the filter aid 9 and suspended on at least a group of particles having a sedimentation velocity higher than that of the cells. Since piping is made so that the fresh medium passed through the filter aid 9 can be transferred to the culture tank 2, the cells suspended on the top of the filter unit 8 are returned to the culture tank 2 together with the fresh medium.

In this case, when the filter aid 9 contains a group of particles having a particle diameter larger than that of the cells, the provision of means such as a screen having an opening size which permits the permeation of the cells but does not permit the permeation of the group of particles on the top of the filter unit 8 enables the filter aid 9 to ascend and mix in the culture tank 2. Further, since the hydrophobic filter membrane 10 is backwashed with the fresh medium, the service life of the membrane can be prolonged.

In the case of a system shown in Fig. 2 wherein a group of particles suspended in the medium is used as the filter aid 9, it is preferred that the fresh medium be fed into the filter unit 8 at such a flow rate that in the filter aid 9 to be developed, a group of particles having the highest ascending velocity descend and the filter aid layer is slightly fluidized. Consequently, the cells separate from the filter aid 9 and settle, and the settled cells are then carried and returned to the culture tank 2 together with a backwash liquid through a pipe connecting the filter unit 8 to the culture tank 2. In this case, it is preferred that a filter 11 having an opening size which permits the permeation of the cells but does not permit the permeation of the filter aid 9 is provided at the bottom of the filter unit 8.

Although there is no particular limitation with respect to the amount of introduction of the fresh medium per operation, the amount of introduction is preferably substantially the same as the amount of discharge of the medium in the above-described medium discharge step, and the flow rate is preferably such that most of the cells captured by the filter aid 9 are returned together with the fresh medium to the culture tank 2.

The stopping of introduction of the fresh medium causes a group of particles of the developing filter aid 9 to settle on or around the filter 10. However, since the filter aid 9 contains a group of particles settleable more rapidly than the cells, the amount of cells which reach the filter 10 is small.

The water-repellent debubbling means is provided for the purpose of breaking bubbles formed on the surface of the liquid medium when a gas is passed through the liquid medium and has a number of openings through which the gas can permeate. It is preferred that the opening ratio and opening diameter of the debubbling means be at least 50% and 50 to 2 mm, respectively. At least the surface layer of the debubbling means is made of a water-repellent material.

The debubbling means exhibits an excellent effect even in the case of the culture of animal cells wherein a serum-containing medium causing particularly remarkable bubbling is used, which enables the oxygen to be supplied with high efficiency through submerged aeration.

The water-repellent material may be a material which exhibits a contact angle of at least 30° when the surface thereof is brought into contact with a droplet of the medium, is substantially insoluble or nondispersible in the medium, and substantially nontoxic to the object cells. The debubbling layer per se may comprise the above-described water-repellent material. Alternatively, it may be prepared by covering, coating, or impregnation through the use of the above-described water-repellent material.

Examples of the water-repellent material include a silane or a sioxane having at least 10 carbon atoms. A water-repellent material having a viscosity of at least $1 \times 10^4$ cP is particularly suitable.

When the viscosity of the water-repellent material is less than $1 \times 10^4$ cP, the surface of the medium is covered with the water-repellent material, or the water-repellent material forms an emulsion with the medium and inhibits the proliferation of the cells through adhesion to the cells. Further, it becomes difficult to separate the product from the medium after completion of the culture.

There is no particular limitation with respect to the upper limit of the number of carbon atoms and the viscosity. However, since the water-repellent material becomes solid when the number of carbon atoms and the viscosity are $1 \times 10^4$ and $1 \times 10^7$ cP, respectively, these values are respective upper limits of the number of carbon atoms and the viscosity.

When the opening ratio is less than 50%, it is difficult to smoothly pass the gas through the debubbling layer when the bubbles present on the surface of the medium are broken, which brings about secondary bubbling. The diameter of the opening of the debubbling layer is preferably 2 to 50 mm. When the diameter exceeds 50 mm, the contact of small bubbles with the debubbling layer becomes insufficient, which makes it difficult to break the bubbles. On the other hand, when the diameter is less than 2 mm, the gas and the medium cannot smoothly go in and out of the opening.

The debubbling layer may have a monolayer structure or a laminated structure. Further, the layer may be in an integral form or a form separable in the longitudinal or lateral direction. The layer structure is properly selected according to the liquid property, the amount of gas flow, and the bubble diameter.

The gas may be usually introduced into the culture tank 2 through a nozzle 4 provided at the bottom of the culture tank. The nozzle may be provided at an appropriate position below the surface of the medium. Further, it may be also provided between the bottom and the surface of the medium. In particular, when a draft tube 3 is provided, the provision of the nozzle 4 under the draft tube 3 contributes to an improvement in the percentage oxygen utilization because the downflow of the medium causes the bubbles to stay in the medium for a longer period of time.

In the present invention, it is preferred that the medium have a surface tension of 45 to $90 \cdot 10^{-5}$ N/cm$^2$ (45 to 90 dyne/cm$^2$). Examples of a highly effective medium include a medium containing a biopolymer such as serum. Besides a serum-containing medium, a medium originally containing or supplied with protein, such as albumin, or nucleic acid is also every effective. Also preferred are a medium containing a component added from the outside and a medium containing a bubbling component secreted during culture.

Examples of the waste component include lactic acid and ammonia contained in a culture filtrate obtained by making use of a hydrophobic membrane.

There is no particular limitation with respect to the means for removing the waste components. For example, the waste components can be removed by a known method through the use of a diffusion dialyzer, an ultrafiltration apparatus, or an electrodialyzer.

However, since the culture filtrate contains a large amount of fine solids, such as serum-derived precipitates and cell debris, it is preferred that the means for removing the waste components have such a form that clogging hardly occurs. Lactic acid and ammonia among the waste components particularly inhibit the proliferation of the cells. For this reason, it is preferred that the removal of the waste components be conducted by making use of ammonia and lactic acid as the barometer of the removal. The culture filtrate also contains large amounts of components necessary for growth of the cells, such as various hormones and proteins. It is desired that these components be recovered and reutilized as much as possible. For this reason, it is preferred that the diffusion dialysis membrane and ultrafiltration membrane each have a fractionation molecular weight of about 1,000 to 10,000. When the fractionation molecular weight of the membrane is less than 1,000, the permeability is so low that the membrane area should be increased and there is a possibility that the membrane cannot be used for a long period of time because of remarkable clogging of the membrane. On the other hand, when this molecular weight exceeds 10,000, substances useful for proliferation of the cells, such as hormones, also permeate through the membrane, which brings about a lowering in the recovery of these substances.

It is preferred that a fine filtration membrane having a pore diameter of about 0.01 $\mu$m be used for separation of useful substances such as protein antibodies having a high molecular weight. It is more advantageous that the separation of the useful substances be conducted through multistage filtration by combining the above-described membranes.

When the waste components are removed by making use of the diffusion dialyzer, it is preferred that a solution having the same concentration and composition as those of low-molecular components contained in the fresh medium such as various liquids, various amino acids and vitamins be used as a dialysis solution.

The solution after removal of the waste components is used as a backwash liquid of the above-described filter unit and then returned to the culture tank to effectively utilize it for culture of the cells.

There is no particular limitation with respect to the biological cells applicable to the present invention, and examples thereof include animal cells, microbial cells, and plant cells. Examples of the animal cells include various cells of vertebrate animals, those of invertebrate animals, and those of protozoa. The cells include not only single cells but also cell clusters.

Examples of the microbial cells include various microorganisms such as bacteria, yeast, mold fungi, and actinomycetes.

Examples of the plant cells include lower plant cells, lower plant cell clusters, algae celle, and algae cell clusters.

With respect to the aeration gas applicable to the present invention, air, oxygen, carbon dioxide, a mixture thereof having an appropriate composition, and a mixture thereof with an inert gas are used according to the applications. In general, an oxygen-containing gas is used for culture of animal cells, while carbon dioxide or a carbon dioxide-containing gas is used for culture of plant cells.

The amount of the cells adhering to the filter membrane comprising a hydrophobic material is smaller than that of the cells adhering to the hydrophilic membrane used in the prior art. Further, since the adherent cells are easily released from the membrane also through backwash, the membrane hardly brings about clogging, which makes it possible to conduct the culture of cells with high efficiency through intermittent backwash.

Further, since effective debubbling of the medium can be conducted by the debubbling means of the present invention, it is possible to efficiently conduct the culture of cells.

The present invention will now be described in more detail with reference to the accompanying drawings.

Fig. 1 is a block diagram of an example of a cell culture system according to the present invention.

In a culture tank 2, cells are cultured in the form of a dispersion or suspension thereof in a medium 1. A nozzle 4 for introducing an oxygen-containing gas into the medium is provided at the bottom of the culture tank. Oxygen necessary for growth of the cells and carbon dioxide for adjusting the pH value of the medium are supplied through the nozzle. Specifically, the flow rates of air from an air compressor 16 and an oxygen-containing gas from a bomb 17 are regulated so that the dissolved oxygen concentration becomes constant, and the flow rate of carbon dioxide from a bomb 18 is regulated so that the pH value becomes 7.0 to 7.6. These gases are passed through a bacterium-removing filter 7 and then blown into the medium through the nozzle 4. The gas blown into the medium fluidizes the medium when it ascends in the form of bubbles towards the surface of the medium. A draft tube 3 is provided for the purpose of efficiently flowing the medium within the tank through the blown gas. The bubbles which have reached the surface of the medium form a bubble layer without being broken. The bubbles can be efficiently broken by bringing the bubble layer into contact with the debubbling layer 5 comprising a hydrophobic material provided on the surface of the medium.

The gas is passed through a mist separator 6 and a filter 7 and then discharged as a waste gas 24 outside the culture tank. Although the culture tank 2 is provided with various devices, such as means for making the dissolved oxygen concentration and the pH value constant, means for sterilizing the inside of the tank and pipe, means for feeding and discharging the medium, means for keeping the temperature constant, sensors for detecting the dissolved oxygen concentration, pH, temperature, internal pressure of the tank, etc., and a sensor for detecting the level of the medium contained in the tank, these devices were not shown in Fig. 1.

A filter unit 8 is provided at the bottom of the culture tank 2. A porous hollow fiber membrane filter 10 is provided within the filter unit 8, and a filter aid 9 is packed on or around the hollow fiber membrane filter 10. The filter aid 9 is supported by a supporting filter 11.

Care should be taken of the hollow fiber membrane filter 10 lest it should be deformed or exposed on the filter aid layer 9 even during the sterilization, backwash, etc. wherein the medium rapidly flows. There is no limitation with respect to the form of the hollow fiber membrane filter.

Figs. 8A and 8B are each an explanatory view of a hydrophobic hollow fiber membrane unit used in the Examples of the present invention. A membrane supporting member 50 is made of a material which exhibits rigidity even at 130°C and has no fear of being attacked by the medium. It has an external shape formed according to the internal shape of the filter unit 8 shown in Fig. 1 and is provided so as not to move within the filter unit even when a slight external force is applied thereto. As shown in Fig. 8A, the filter supporting member 50 is provided with a number of small holes 55 adapted for supporting the hollow fiber membrane. The size of the holes is preferably such that the hollow fiber membrane 10 can be freely passed therethrough. Fig. 8B is a filtration unit comprising a membrane supporting member 50 and a hollow fiber membrane 10 mounted thereon. Although the hollow fiber membrane 10 may constitute the filtration unit in the form of a single hollow fiber membrane, it is usually preferred that the filtration unit comprise a plurality of hollow fiber membranes. In this case, the bundling of a plurality of hollow fiber membranes with a membrane connecting member 51 makes it easy to mount the filtration unit on the filtration unit.

Figs. 9A and 9B are each a schematic view of the membrane connecting member 51. This membrane connecting member is useful for jointing a hollow fiber membrane made of a material causing a difficulty in jointing the membrane through bonding or welding, e.g., a hollow fiber membrane made of a tetrafluoroethylene resin.

Fig. 9A is a diagram of a surface of the membrane connecting member 51 opposite to the surface to which the hollow fiber membranes are connected. A membrane expansion member 52 is enclosed in the membrane connecting member 51. It is preferred that the external surface of the membrane connecting member 51 is formed into a circular shape. Fig. 9B is a cross section taken along line A-A' of Fig. 9A. The membrane expansion member 52 has an inner diameter substantially the same as that of the hollow fiber membrane, an outer diameter larger than the inner diameter of the hollow fiber membrane, a top portion tapered so as to be easily inserted into the hollow fiber membrane, and a partially protruding portion adapted for preventing release of the hollow fiber membrane. Each portion of the hollow fiber membranes having the membrane expansion member 52 inserted thereinto is enclosed by the membrane connecting member 51, and the membrane connecting member is then cured to firmly bind the hollow fiber membranes. There is no particular limitation with respect to the membrane connecting member as far as it has no adverse effect on the cells, causes no corrosion in the medium, and can withstand steam sterilization at about 130°C. For example, a two-pack curable epoxy resin may be used.

The medium is replaced by the following method. Valves 31 and 32 shown in Fig. 1 are closed, and a valve 33 is opened. A pump 22 is actuated to lead the medium 1 from the culture tank 2 to the filter unit 8. The cells contained in the medium are captured by the surface of each of the filter aid 9 and the porous hollow fiber filter 10 during passing of the medium through the filter aid and the hollow fiber filter. In this case, a porous hollow fiber filter 10 having an opening size which does not permit the permeation of the particles of the filter aid 9 and the cells was selected. A culture filtrate obtained by filtration of the cells is stored in a culture filtrate storage tank 15.

After a predetermined amount of the medium is discharged from the culture tank 2, the pump 22 is stopped, and the valve 33 is closed. Then, the valves 31 and 32 are opened, and the pumps 19, 20 are actuated. The pumps 19 and 20 feed a fresh medium from a fresh medium storage tank 12 into the filter unit. At that time, the pump 19 feeds the fresh medium through the bottom of the filter unit 8 at such a flow rate that the packed bed of the filter aid 9 is floated and developed. On the other hand, the pump 20 reversely feeds the fresh medium under pressure to the porous hollow fiber membrane 10 for backwash, thereby releasing the solids, such as cells, adhering to the surface of the filter 10. The total flow rate of the pumps 19 and 20 is regulated so that no filter aid 9 dissipates from around the filter unit.

The cells captured on the surface of each of the filter aid 9 and the hollow fiber membrane filter 10 in the above-described step of filtration are released from the packed bed through the development of the filter aid 9. Since the specific gravity, particle diameter, and form of the particles of the filter aid 9 are selected so that the particles of the filter aid 9 have a higher sedimentation velocity than that of the cells, the cells ascend on the developed packed bed and then returned to the culture tank 2 together with the fresh medium.

Fig. 2 is a block diagram of another example of the cell culture system according to the present invention. The characteristic feature of the present example resides in that a particle having a smaller specific gravity than that of the medium is used as the filter aid 9 for the filter unit 8. Examples of the filter aid 9 include hollow glass beads and hollow carbon heads. The filter aid 9 is supported by two supporting filters 11 respectively provided at the top and bottom of the filter unit 8. The opening size of the supporting filter 11 is selected so that no particle of the filter aid 9 can permeate through the opening while the cells can freely permeate through the opening.

According to the present example, the cells captured by the packed bed of the filter aid 9 and the hollow fiber membrane filter 10 during backwash can be more completely returned to the culture tank.

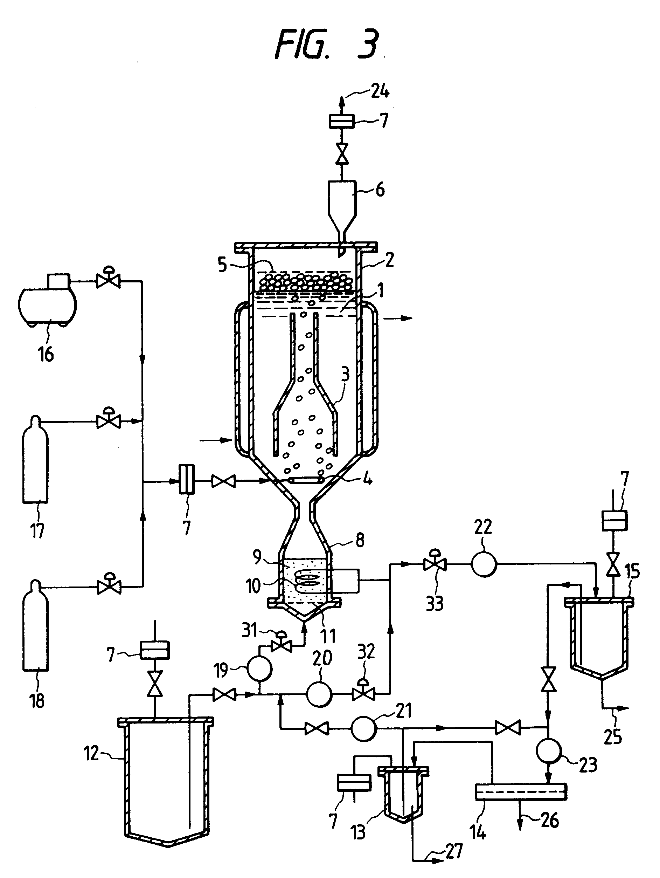

Fig. 3 is a block diagram of a further example of the cell culture system according to the present invention. The characteristic feature of the present example resides in that means for removing waste components was added to the example shown in Fig. 1. In the present example, ultrafiltration is used as the means for removing waste components.

An ultrafilter 14 is connected to the culture filtrate storage tank 15 through a pipe. An ultrafiltration membrane having a fractionation molecular weight of 10,000 is used for the ultrafilter 14. This membrane permits the permeation of waste components, such as lactic acid and ammonia, but does not permit the permeation of substances, such as hormones, useful for growth of the cells. The culture filtrate is fed under pressure through a pump 23 into the ultrafilter 14. Low-molecular substances, such as waste components, permeate through the ultrafiltration membrane during the contact of the culture filtrate with the membrane and is discharged as waste components 26 outside the system.

The liquid from which the waste components have been removed with the ultrafilter 14 is stored in a waste component-free liquid storage tank 13. Since the amount of the liquid to be filtered by the ultrafiltration is small, the filtration is conducted by circulating the liquid between the ultrafilter 14 and the waste component-free liquid storage tank with the pump 23 to remove the waste components. In general, it is necessary that when the waste component-free liquid is reutilized as part of the fresh medium, the waste component concentration be 1000 ppm or less, preferably 100 ppm or less for lactic acid and 20 ppm or less, preferably 5 ppm or less for ammonia.

The waste component-free liquid is added to the fresh medium as part of the backwash liquid of the filter unit 8 by actuating a pump 21, returned to the culture tank 2, and utilized again for the proliferation of the cells. Since the waste component-free liquid contains not only large amounts of useful components, such as hormones and growth factors, remaining in the culture filtrate, but also growth promotion factors and useful products secreted from the cells per se, the use thereof gives a good result with respect to the growth of the cells.

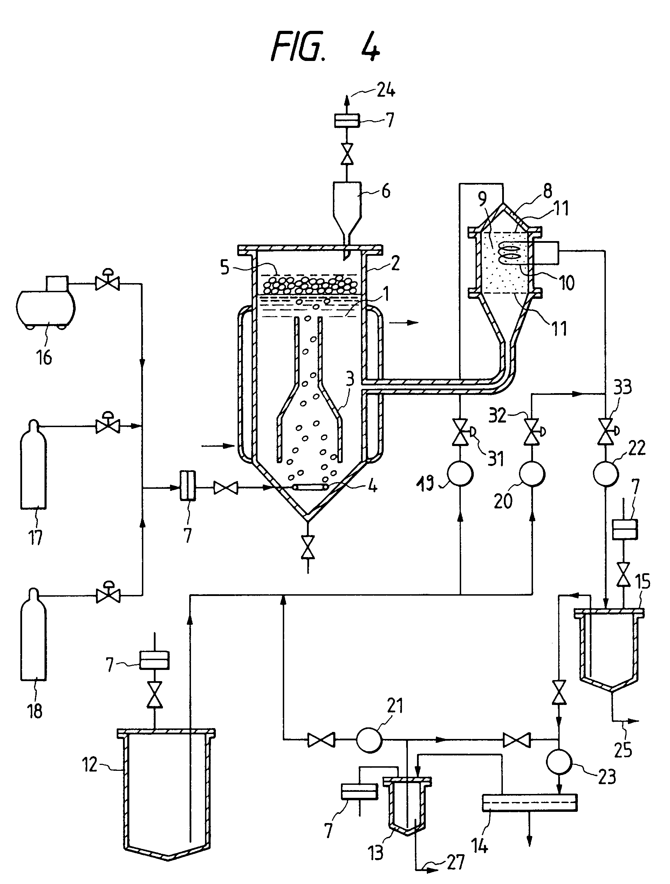

Fig. 4 is a block diagram of a further example of the cell culture system according to the present invention.

The characteristic feature of the present example resides in that in the example shown in Fig. 3, a particle having a smaller specific gravity than that of the medium is used as the filter aid 9 for the filter unit 8. Examples of the filter aid 9 include hollow glass beads and hollow carbon beads. The filter aid 9 is supported by two supporting filters 11 respectively provided at the top and bottom of the filter unit 8. The opening size of the supporting filter 11 is selected so that no particle of the filter aid 9 can permeate through the opening while the cells can freely permeate through the opening. The other structure is the same as that of the example shown in Fig. 3.

According to the present example, the cells captured by the packed bed of the filter aid 9 and the hollow fiber membrane filter 10 during backwash can be more completely returned to the culture tank.

Fig. 5 is a block diagram of a further example of the cell culture system according to the present invention.

The characteristic feature of the present example resides in that in the example shown in Fig. 3, the hydrophobic membrane 10 is provided at the upper part of the culture tank 2 and an agitator is additionally provided. In the present example, the medium is replaced as follows. A valve 33 is opened, and a valve 32 is closed. When a pump 22 is actuated, the inside of the hollow fiber membrane is evacuated. As a result, the cells suspended in the medium are captured on the surface of the hollow fiber membrane, and a culture filtrate free from the cell is extracted within the membrane and stored in the culture filtrate storage tank 15.

After a predetermined amount of the medium is discharged from the culture tank 2, the pump 22 is stopped, and the valve 33 is closed. Then, the valve 32 is opened, and pumps 20 and 21 are actuated. The pump 20 feeds the fresh medium from the fresh medium storage tank 12, while the pump 21 feeds the waste component-free liquid from the waste component-free liquid storage tank 13. The fresh medium and the waste component-free liquid are fed under pressure into the hollow fiber membrane and flowed through the pores on the surface of the membrane into the culture tank 2. At that time, solids, such as cells, adsorbed on the outer surface of the hollow fiber membrane and within the pores are released to regenerate the filtration surface. In order to prevent the cells and contaminants from depositing on the surface of the hydrophobic membrane 10, it is preferred that the hydrophobic membrane 10 be provided at a place where the medium flows at the highest speed. As demonstrated in the present example, when an agitator 29 is provided and a hydrophobic membrane is provided around the agitator, the flow of the medium delivered by the rotation of the agitation blade causes the solids present on the surface of the membrane to move without staying, so that the deposition of the solids on the surface of the membrane can be effectively prevented.

There is no particular limitation with respect to the form of the filtration unit used in the present example. However, the form of the filtration unit is preferably one which does not inhibit the flow of the medium and applies no unnecessary external force to the cells.

The filtration units shown in Figs. 10 to 13 each have a form particularly suitable for the present example.

According to the present invention, since the filtration is conducted in the culture tank, there occur no phenomena such as lack of oxygen and lowering in the temperature during filtration, which enables the cells to be proliferated under good conditions.

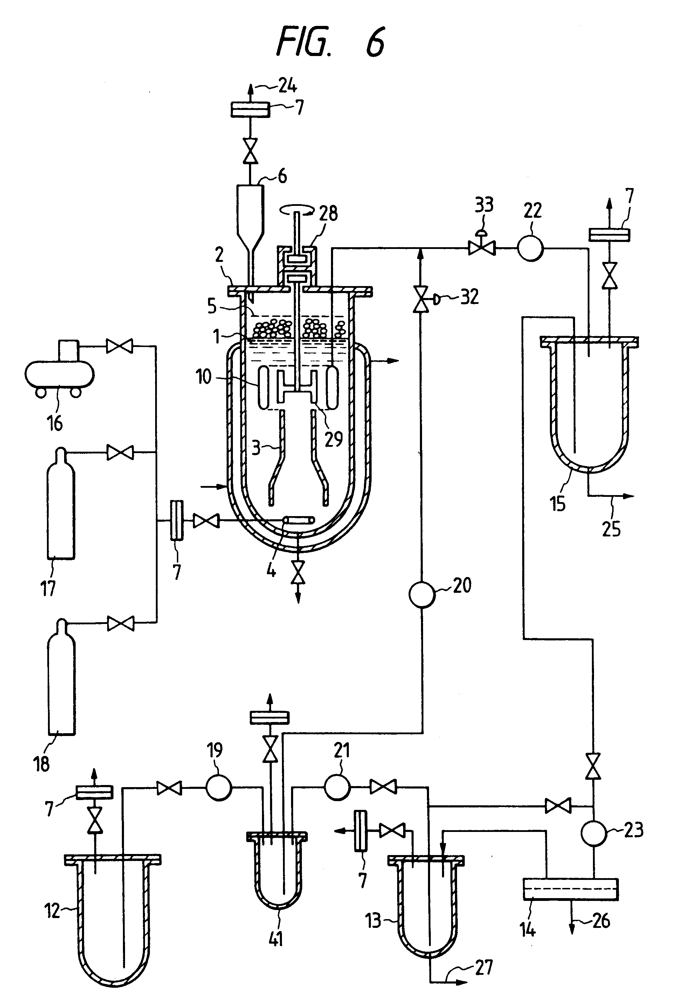

Fig. 6 is a block diagram of a further example of the cell culture system according to the present invention. The characteristic feature of the present example resides in that a medium preparation tank 41 is added to the example shown in Fig. 5.

The backwash liquid used for backwash of the hydrophobic hollow fiber membrane provided within the culture tank is prepared by preliminary mixing the fresh medium with the waste component-free liquid in the medium preparation tank 41. The medium preparation tank 41 is provided with means for measuring the concentrations of lactic acid, ammonia, and protein. By this means, the mixing ratio of the fresh medium to the waste component-free liquid is adjusted.

According to the present example, a medium suitable for the proliferation of cells can be always used to conduct the culture.

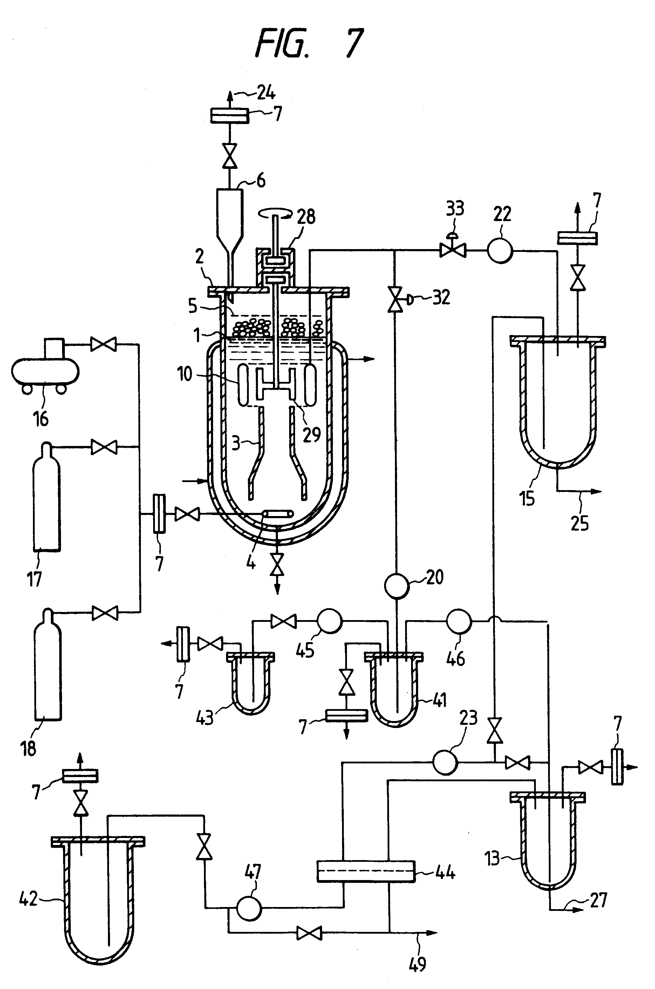

Fig. 7 is a block diagram of a further example of the cell culture system according to the present invention. The characteristic feature of the present example resides in that a diffusion dialyzer is used as the water component removing device instead of the ultrafiltration membrane in the example shown in Fig. 6.

The culture filtrate is led through a pipe to a diffusion dialyzer 44 and circulated through a pump 23 between a waste component storage tank 13 and the diffusion dialyzer 44.

A low-molecular component solution containing, dissolved therein, various low-molecular components, such as amino acids, vitamins, inorganic salts, glucose, etc. is stored in a low-molecular component solution storage tank 42 and transferred through a pump 47 to the diffusion dialyzer. In the diffusion dialyzer 44, the culture filtrate is brought into contact with the low-molecular component solution via a dialysis membrane. The low-molecular components passable through the dialysis membrane are dialyzed during the above-described contact. Specifically, waste components including lactic acid and ammonia contained in the culture filtrate are diffused in the low-molecular component solution, while various amino acids, glucose, various inorganic salts, and vitamins dissolved in the low-molecular component solution are diffused in the culture filtrate. High-molecular components, such as various hormones and growth factors, useful for the proliferation of cells remain as such in the waste component-free liquid without being dialyzed. The dialysis is conducted in such a manner that the lactic acid concentration and the ammonia concentration are 1000 ppm or less, preferably 100 ppm or less and 20 ppm or less, preferably 5 ppm or less, respectively.

The waste component-free liquid from which the waste components have been removed by diffusion dialysis is transferred to the medium preparation tank 41 through the pump 46. A high-molecular component solution is fed from a high-molecular component solution storage tank 43 through a pump 45 to the medium preparation tank, where the high-molecular component solution is mixed with the above-described waste component to prepare a fresh medium. High-molecular components, such as serum and hormones, which cannot permeate through the dialysis membrane are dissolved in the high-molecular component solution. The medium preparation tank 41 is provided with means for means for measuring the concentrations of lactic acid, ammonia, and protein. By this means, the mixing ratio of the high-molecular component solution to the waste component-free liquid is adjusted.

According to the present invention, a medium suitable for the proliferation of cells can be always prepared, so that it is possible to culture the cells in a high concentration.

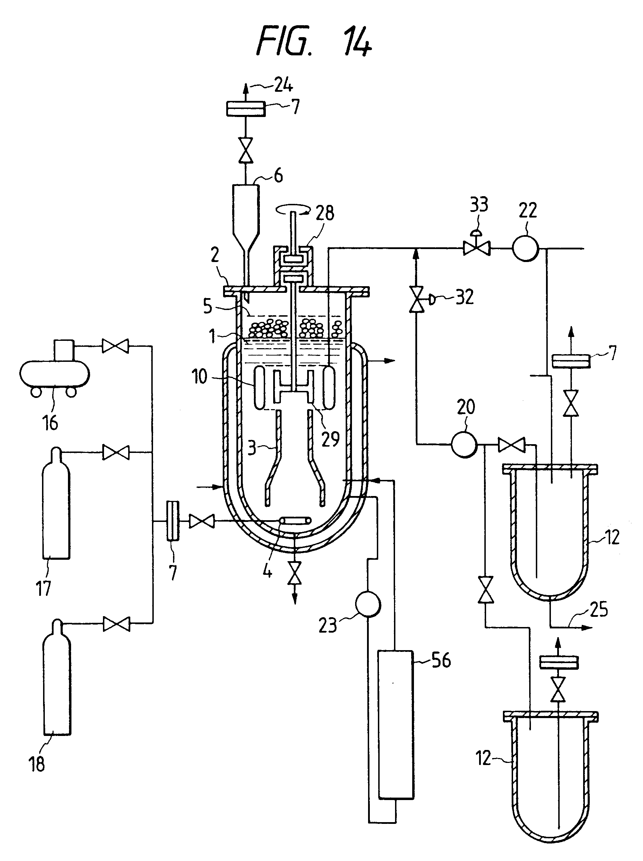

Fig. 14 is a further example of the present invention wherein the means for removing waste components shown in Fig. 6 is replaced with an adsorption cylinder.

Zeolite was immobilized on an insoluble carrier for the adsorption of ammonia, while lactate dehydrogenase was immobilized on an insoluble carrier for the adsorption of lactic acid. Adsorption and regeneration can be conducted regardless of the step of culture by providing a plurality of adsorption cylinders and properly switching pipes. According to the present example, since only the waste components can be selectively removed, the amount of loss of the medium is small.

The present invention will now be described in more detail with reference to the following Examples.

Example 1 (not covering the invention)

A SUS316 membrane expansion member 52 shown in Fig. 9 and having a maximum outer diameter of 2.5 mm, an inner diameter of 0.9 mm, and a length of 30 mm was inserted into both ends of a hydrophobic hollow fiber membrane (TB-21) comprising a terafluoroethylene resin and having an outer diameter of 2 mm, an inner diameter of 1 mm, a pore diameter of 0.8 $\mu$m, and a length of 1 m. Then 4 hollow fiber membranes of the type as described above were put in order and inserted into a cylinderical curing mold having an inner diameter of 15 mm and a depth of 30 mm. A two-pack curable epoxy resin (Araldite Rapid; a registered trademark of a product of Ciba-Geigy) was put in the above-described curing mold immediately after mixing of a main agent and a curing agent of the epoxy resin, and a centrifugal force of 1,000 G was applied by making use of a centrifugal separator, thereby curing the epoxy resin during application of the centrifugal force. After the epoxy resin had been completely cured, the cured product was taken out of the curing mold, and the end of the cured product was cut by 5 mm.

Steam having a pressure of 1.8 kg/cm$^2$G and a temperature of 130°C was fed from both ends of the above-formed hollow fiber membrane unit, and the hollow fiber membrane unit was maintained in this state for 1 hr. No hollow fiber membrane was pulled out of the membrane connecting member 51 even when a tensile load of 1 kg per hollow fiber membrane was applied after the hollow fiber membrane unit was cooled to room temperature.

Comparative Example 1

A hollow fiber membrane unit was prepared in the same manner as that of Example 1, except that the hollow fiber membranes were fixed with an epoxy connecting agent without inserting the membrane expansion member 52 into both ends of the hollow fiber membranes. Steam having a pressure of 1.8 $kg/cm^2G$ and a temperature of 130°C was fed from both ends of the above-formed hollow fiber membrane unit, and the hollow fiber membrane unit was maintained in this state for 1 hr. After the hollow fiber membrane unit was cooled to room temperature, a tensile load of 1 kg per hollow fiber membrane was applied. This caused the hollow fiber membranes to be pulled out of the membrane connecting member 51.

Example 2

15 flat flasks each containing 5 ml of a medium comprising 9.4 g/l of Eagle MEM Medium (Eagle MEM Nissui (1); a product of Nissui Pharmaceutical Co., Ltd.), 0.292 g/l of glutamin, 29 ml/l of a 7.5% aqueous sodium bicarbonate solution, 20 g/l of glucose, and 100 ml/l of fetal calf serum and, inoculated thereon, a tumor cell line of a rat liver, JTC-1 (Japan Tissue Culture No. 1) were subjected to stationary culture. The culture was conducted at 37°C, and air was used as a gas for constituting a gas phase.

After culture for 3 days, the cells adhering to the surface of each flask were released, thereby preparing 75 ml of a medium having a cell concentration of $5.1 \times 10^5$/ml. This medium was centrifuged, and the supernatant was discarded. The residue was suspended in an equal volume of a fresh medium.

Subsequently, a roller bottle having a diameter of 110 mm, a length of 171 mm, and a volume of 1,250 ml was charged with 75 ml of the above-described cell suspension and 175 ml of the above-described fresh medium, and the mixture was cultured at 37°C and 2 rpm for 3 days by making use of air as a gas for constituting the gaseous phase. Then the medium was centrifuged, and the supernatant was discarded. The residue was suspended in an equal volume of a fresh medium. The suspension was cultured again under the above-described conditions for 3 days. The medium was centrifuged, and the supernatant was discarded. The residue was suspended in a 2-fold volume of a fresh medium. The suspension was divided into a 2-fold number of roller bottles, and cultured again under the above-described conditions for 3 days.

The above-described procedures were repeated twice to prepare 2,000 ml of a seed medium having a cell concentration of $1.7 \times 10^6$/ml. This medium was centrifuged, and the supernatant was discarded. The residue was suspended in an equal volume of a fresh medium.

Then a cylindrical stainless culture tank having a diameter of 165 mm, a height of 350 mm, and a volume of 7,000 ml in the cell culture system shown in Fig. 1 was charged with 2,000 ml of the above-described seed cell suspension and 3,000 ml of a medium (5,000 ml in total), and the mixture was maintained at 37°C. A stainless steel net provided with square meshes (length of a side: 3 mm) and having a surface thinly coated with a silane, an organic silicon polymer (viscosity: $1 \times 10^5$ cP), was placed 50 mm above the surface of the liquid, and the mixture was cultured by introducing an oxygen-containing gas through a ring sparger having a diameter of 60 mm (a hole diameter of 1 mm; 10 holes) and provided at the bottom of the tank. The amount of introduction of the oxygen-containing gas was adjusted with a dissolved oxygen concentration adjustor capable of automatically adjusting the amount of air and oxygen so that the dissolved oxygen concentration (DO) of the medium was 2.5 ppm. Further, carbon dioxide was added to the oxygen-containing gas and introduced into the medium with a pH adjustor so that the pH value of the medium was 7.0 to 7.6.

A filter unit is connected to the bottom of the culture tank through a pipe. A supporting filter is provided at the bottom of the filter unit to support a hydrophilic filter aid layer. A hydrophobic hollow fiber filter unit is buried in the hydrophilic filter aid layer. The hydrophobic hollow fiber filter unit was prepared to have a form shown in Fig. 8B by making use of 4 hydrophobic hollow fiber membranes each having a length of 1.2 m according to the method described in Example 1. A product prepared by treating a glass bead having a diameter of 70 μm with ethyl alcohol to impart a hydrophilic nature to the glass bead was used as the hydrophilic filter aid.

The inside of the hollow fiber membrane filter was evacuated with a peristaltic pump for withdrawal to withdraw the culture filtrate at a flow rate of 20 ml/min. The mixing of the cells in the cell filtrate was not observed at all. When the amount of withdrawal reached 500 ml, the peristaltic pump for withdrawal was stopped. The backwashing of the hollow fiber membrane filter and the development of the filter aid layer were conducted with a peristaltic pump for backwashing of a hollow fiber membrane filter and a peristaltic pump for development of a filter aid layer, respectively.

The hollow fiber membrane filter was backwashed at a flow rate of 80 ml/min for 2 min. The filter aid layer was developed at a flow rate of 70 ml/min during backwashing of the hollow fiber membrane filter and at a flow rate of 150 ml/min after the completion of backwashing of the hollow fiber membrane filter, and the development was controlled so as to be completed when the amount of the fresh medium fed into the culture tank reached the same amount as that of withdrawal. A fresh medium prepared in the fresh medium storage tank was used for the backwashing of the hollow fiber membrane filter and the development of the filter aid. The filtration of the medium, the backwashing of the hollow fiber membrane filter and the development of the filter medium layer were conducted ten times per day so that the medium contained in the culture tank is replaced once a day.

The results of the present Example are shown in Fig. 16. After 12 days from the initiation of the culture, it was possible to maintain a cell concentration of at least $1 \times 10^7$/ml and a survival rate of at least 88%. The maximum cell concentration was $1.6 \times 10^7$/ml.

Comparative Example 2

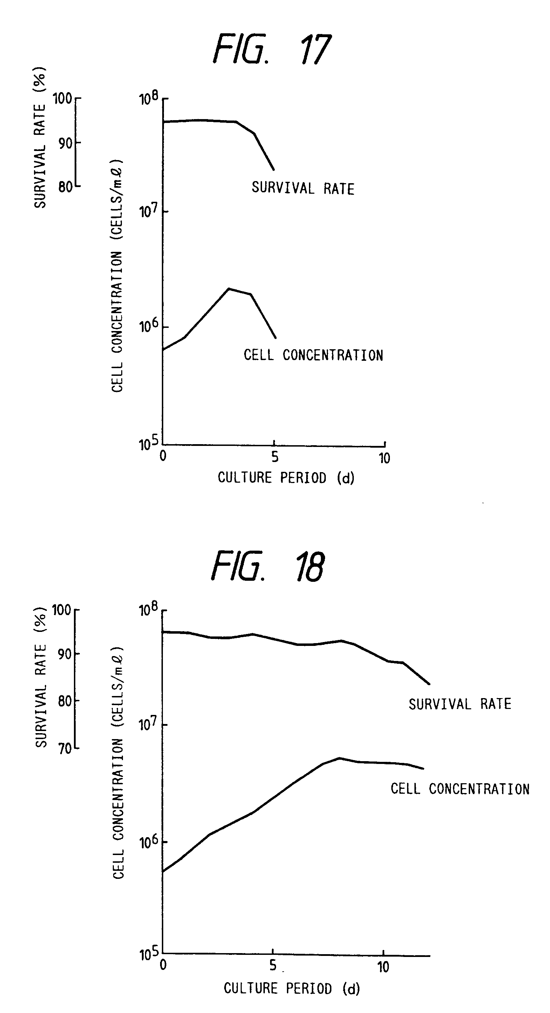

Culture was conducted in the same manner as that of Example 2, except that a culture system shown in Fig. 1 from which the filter unit, the hollow fiber membrane filter, and the filter aid had been removed was used. The results are shown in Fig. 17. The replacement of the medium could not be conducted due to the removal of the filter unit, the hollow fiber membrane filter, and the filter aid. This brought about depletion of nutrient components, so that the cell concentration rapidly decreased after it reached $2.1 \times 10^6$/ml. This clearly substantiates that the replacement of the medium is indispensable for culture of cells.

Comparative Example 3

Culture was conducted in the same manner as that of Example 2, except that in the culture system shown in Fig. 1, the supply of the oxygen was conducted by liquid surface aeration. The results are shown in Fig. 18. The maximum cell concentration was $5.6 \times 10^6$/ml, and the survival rate gradually lowered. This shows that the medium lacks in the oxygen necessary for the proliferation of the cells. Thus it is apparent that no sufficient amount of oxygen can be fed by the liquid surface aeration.

Comparative Example 4

Culture was conducted in the same manner as that of Example 2, except that a culture system shown in Fig. 1 from which the debubbling layer had been removed was used. In this culture, bubbles flowed out from a culture waste gas pipe. This substantiates that in order to feed oxygen through submerged aeration, it is necessary to provide debubbling means within the culture tank.

Example 3

A seed cell suspension having a cell concentration of $1.8 \times 10^6$/ml was prepared in the same manner as that of Example 2. Subsequently, culture was conducted in a cell culture system shown in Fig. 6. The culture tank in this system was a SUS316 cylindrical tank having an internal volume of 7,000 ml, and an agitator 29 was provided for the purpose of allowing the medium to flow. A hydrophobic hollow fiber membrane filter unit 10 for separating the cells from the medium had a form shown in Fig. 13 and was provided around the surface of revolution of the agitating blade. The hydrophobic hollow fiber membrane filter unit was prepared by making use of 4 hollow fiber membrane filters each having a length of 1.5 m and the same type as that used in Example 1. The means for feeding oxygen, the debubbling means, and culture conditions were the same as those of Example 2.

The culture tank was charged with 2,000 ml of the above-described cell suspension and 3,000 ml of the same medium as that of Example 2 (5,000 ml in total), and the mixture was maintained at 37°C. Batch culture was conducted for two days after the initiation of the culture, and the replacement of the medium was started from three days after the initiation of the culture. The inside of the hollow fiber membrane filter was evacuated with a peristaltic pump to withdraw the culture filtrate at a flow rate of 10 ml/min. The mixing of the cells in the cell filtrate was not observed at all. When the amount of withdrawal reached 500 ml, the peristaltic pump for withdrawal was stopped. Then a fresh medium was fed under pressure into the hollow fiber membrane filter 10 at a flow rate of 200 ml/min with a peristaltic pump for backwashing of the hollow fiber membrane filter, thereby backwashing the hollow fiber membrane filter 10. The medium used for the

EP 0 336 966 B1

backwashing comprised 9.4 g/l of Eagle MEM Medium, 0.292 g/l of glutamin, 29 ml/l of a 7.5% aqueous sodium bicarbonate solution, and 50 ml/l of fetal calf serum and was stored in the fresh medium storate tank 12. The backwashing was controlled so as to be completed when the amount of the fresh medium fed into the culture tank reached the same amount as that of withdrawal of the culture filtrate. The filtration and the backwashing were conducted ten times per day so that the medium contained in the culture tank is completely replaced once a day with the fresh medium.

The withdrawn culture filtrate was stored in the culture filtrate storage tank 15. Thereafter, low-molecular components containing waste components were filtered through an ultrafilter 14, thereby concentrating high-molecular components. The high-molecular components were stored in the waste component-free liquid storage tank 13. The waste component-free liquid was circulated between the ultrafilter 14 and the waste component-free liquid storage tank 13 through a peristaltic pump until the volume of the waste component-free liquid was 1/10 of the culture filtrate. The ultrafilter had a fractionation molecular weight of 10,000.

From eight days after the initiation of the culture, the fresh medium was mixed with the waste component-free liquid in the medium preparation tank in a proportion of 900 ml of the fresh medium per 100 ml of the waste component-free liquid. The mixture was used for the backwashing of the hollow fiber membrane filter 10 in the medium preparation tank.

From 18 days after the initiation of the culture, the filtration and the backwashing were conducted 20 times per day so that the medium contained in the culture tank was replaced twice a day.

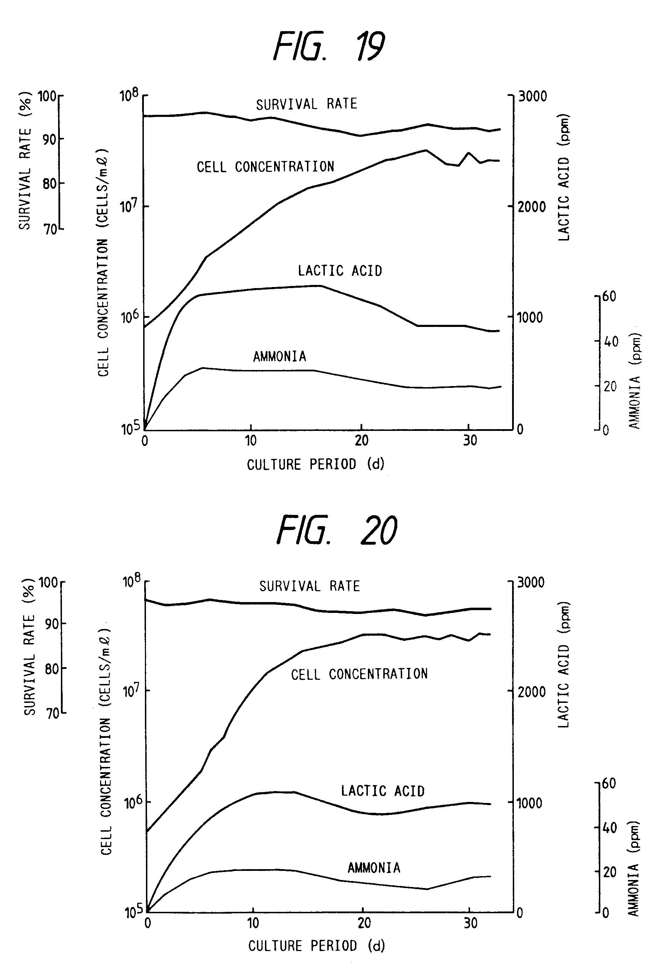

The results are shown in Fig. 19. By virtue of the removal of the waste components through the ultrafilter, the lactic acid and ammonia concentrations were lower than those attained in Example 2. Further, the maximum cell concentration reached $2.8 \times 10^7$/ml, which substantiates that the present Example has an excellent effect.


Example 4

A seed cell suspension having a cell concentration of $1.1 \times 10^6$/ml was prepared in the same manner as that of Example 2. Subsequently, culture was conducted in a cell culture system shown in Fig. 7. This system corresponds to the culture system shown in Fig. 6 and used in Example 3 wherein only the means for removing the waste components was replaced by the diffusion dialyzer 44. The means for feeding oxygen, the debubbling means, the means for replacing the medium, and the culture conditions were the same as those of Example 3.

The culture tank was charged with 2,000 ml of the above-described cell suspension and 3,000 ml of the same medium as that of Example 2 (5,000 ml in total), and the mixture was maintained at 37°C. Batch culture was conducted for two days after the initiation of the culture, and the replacement of the medium was started from three days after the initiation of the culture. The inside of the hollow fiber membrane filter 10 was evacuated with a peristaltic pump to withdraw the culture filtrate at a flow rate of 15 ml/min. The withdrawn culture filtrate was stored in the culture filtrate storage tank 15. The mixing of the cells in the cell filtrate was not observed at all. When the amount of withdrawal reached 500 ml, the peristaltic pump for withdrawal was stopped.

The culture solution was fed from the culture filtrate storage tank 15 to the diffusion dialyzer 44 with a peristaltic pump to remove low-molecular components containing waste components through the diffusion dialysis. A membrane which permits permeation of a substance having a molecular weight of 6,000 or less but does not permit permeation of a substance having a molecular weight exceeding 6,000 was used as the diffusion dialysis membrane. In the diffusion dialysis, a low-molecular component solution comprising 9.5 g/l of Eagle MEM Medium, 0.292 g/l of glutamin, 29 ml/l of a 7.5% aqueous sodium bicarbonate solution, and 20 ml/l of glucose was used as a control and fed at a flow rate twice higher than that of the culture filtrate. The waste component-free liquid from which low-molecular components containing waste components had been removed was stored in the waste component-free liquid storage tank.

In the medium preparation tank, fetal calf serum was added in an amount of 1% to the waste component-free liquid. The mixture was fed under pressure through a peristaltic pump into the hollow fiber membrane filter 10 at a flow rate of 200 ml/min to backwash the hollow fiber membrane filter 10. The backwashing was controlled so as to be completed when the amount of the backwash liquid fed into the culture tank reached teh same amount as that of withdrawal of the culture filtrate. The filtration and the backwashing were conducted 30 times a day so that the medium contained in the culture tank is replaced three times a day.

The results of the present Example are shown in Fig. 20. By virtue of the removal of the waste components through diffusion dialysis, the lactic acid and ammonia concentrations were lowered, so that the maximum cell concentration reached $3.0 \times 10^7$/ml.

14

Comparative Example 5

Culture of cells was conducted in the same manner as that of Example 2, except that in the culture apparatus shown in Fig. 1, a hydrophilic filter membrane (a product of Amicon Corporation; Diaflow Hollow Fiber System; DH-l; hollow fiber membrane; HIMPOI-43; a pore diameter of 1.1 $\mu$m) was used instead of the hydrophobic filter membrane.

When the filter membrane was backwashed, the filtration flow rate was rapidly lowered, and clogging occurred 6 days after the initiation of the culture, which made it impossible to continue the culture.

Example 5

15 flat flasks each containing 5 ml of the following medium and, inoculated thereon, a tumor cell line of a rat liver, JTC-1 (Japan Tissue Culture No. 1) were subjected to stationary culture.

The culture was conducted at 37°C, and a gaseous phase gas comprised air containing 5% carbon dioxide mixed therein.

|                     | medium composition |        |
|---------------------|---------------------|--------|
| glucose             | 1000                | mg/l   |
| glutamine           | 200                 | "      |
| aspartic acid       | 100                 | "      |
| glutamic acid       | 50                  | "      |
| glycine             | 10                  | "      |
| alanine             | 10                  | "      |
| leucine             | 20                  | "      |
| isoleucine          | 20                  | "      |
| valine              | 15                  | "      |
| arginine            | 10                  | mg     |
| cystine             | 10                  | "      |
| cysteine            | 10                  | "      |
| serine              | 20                  | "      |
| threonine           | 15                  | "      |
| lysine              | 10                  | "      |
| methionine          | 10                  | "      |
| sodium citrate      | 25                  | "      |
| citric acid         | 10                  | "      |
| $\alpha$-ketoglutaric acid | 15           | "      |
| biotin              | 0.1                 | "      |
| thiamine            | 0.1                 | "      |
| pyridoxal           | 0.01                | "      |
| nicotinic acid      | 0.01                | "      |
| riboflavin          | 0.02                | "      |
| inositol            | 0.1                 | "      |
| vitamin $B_{12}$    | 0.001               | "      |
| glutathione         | 1                   | "      |

| | | |
|---------------------|------|------|
| sodium chloride     | 50   | mg   |
| potassium chloride  | 50   | "    |
| magnesium chloride  | 10   | "    |
| calcium chloride    | 10   | "    |
| manganese chloride  | 0.01 | "    |
| sodium bicarbonate  | 10   | "    |
| fetal calf serum    | 100  | ml   |
| pH 7.0     total    | 1 litter | |

16

After culture for 3 days, the cells adhering to the surface of each flask were released, thereby preparing 75 ml of a seed medium having a cell concentration of 1.1 x 10⁵/ml. This medium was centrifuged, and the supernatant was discarded. The residue was suspended in an equal volume of a fresh medium.

Then a cylindrical glass culture tank having a diameter of 70 mm, a height of 390 mm, and a volume of 1,500 ml was charged with 75 ml of the above-described seed cell suspension and 725 ml of a liquid medium, and 10 ml of fetal calf serum (900 ml in total), and the mixture was maintained at 37°C. A stainless steel net provided with square meshes (length of a side: 5 mm) and having a surface thinly coated with a silane, an organic silicon polymer (HIVAC-G; a product of The Shin-Etsu Chemical Co., Ltd.; a viscosity of 1 x 10⁵ cP), was placed 10 mm above the surface of the medium, and the mixture was cultured for 5 days while introducing aseptic air through a single-hole nozzle 4 having an inner diameter of 1 mm and provided at the bottom of the tank 2 at a flow rate of 0.2 cm/sec. The oxygen travel speed at an air feed rate of 0.2 cm/sec was 7 mmol $O_2$/1•hr. In the culture, the cell concentration reached 6 x 10⁶/ml. The concentrations of ammonia and lactic acid produced during the culture were 40 ppm and 1100 ppm, respectively. The dissolved oxygen concentration was maintained at 2 to 5 ppm during the culture. Bubbles formed during the culture were brought into contact with the debubbling layer 5 and smoothly broken. Therefore, no bubbles overflowed from the upper surface of the debubbling layer into the gaseous phase of the tank throughout the culture.

Example 6

15 flat flasks each containing 5 ml of the following medium and, inoculated thereon, a tumor cell line of a rat liver, JTC-1 (Japan Tissue Culture No. 1) were subjected to stationary culture. The culture was conducted at 37°C, and a gaseous phase gas comprised air containing 5% (V/V) carbon dioxide mixed therein.

|  | medium composition | |
| --- | --- | --- |
| glucose | 2000 | mg/l |
| arginine hydrochloride | 126 | " |
| cystine | 24 | " |
| glutamine | 292 | " |
| histidine hydrochloride | 42 | " |
| isoleucine | 52 | " |
| leucine | 52 | " |
| lysine hydrochloride | 73 | " |
| methionine | 15 | " |
| phenylalanine | 32 | " |
| threonine | 48 | " |
| tryptophan | 10 | " |
| tyrosine | 36 | " |
| valine | 46 | " |
| thiamin | 1.0 | " |
| riboflavin | 0.1 | " |
| pyridoxal | 1.0 | " |
| pantothenic acid | 1.0 | " |
| nicotinic acid | 1.0 | " |
| biotin | 0.02 | " |

17

| choline hydrochloride | | 1.8 | mg/l |
|---|---|---|---|
| folic acid | | 1.0 | " |
| inositol | | 2.0 | " |
| sodium chloride | | 8000 | " |
| potassium chloride | | 200 | " |
| disodium hydrogenphosphate dodecahydrate | | 200 | " |
| fetal calf serum | | $1 \times 10^5$ | |
| pH 7.0 | total 1 litter | | |

After culture for 3 days, the cells adhering to the surface of each flask were released, thereby preparing 75 ml of a seed medium having a cell concentration of $1.21 \times 10^5$/ml. This medium was centrifuged, and the supernatant was discarded. The residue was suspended in an equal volume of a fresh medium.

Then a cylindrical glass culture tank having a diameter of 70 mm, a height of 390 mm, and a volume of 1,500 ml was charged with 75 ml of the above-described seed cell suspension and 725 ml of a liquid medium, and 10 ml of fetal calf serum (900 ml in total), and the mixture was maintained at 37°C. A polyethylene honeycomb debubbling layer 5 having a height of 5 mm (one side of regular hexagonal hole: 4 mm) and having a surface thinly coated with a polysiloxane (HIVAC-G; a product of The Shin-Etsu Chemical Co., Ltd.) was placed 0.5 mm above the surface of the medium. The mixture was cultured for 5 days while introducing sterilized air through a porous glass nozzle 4 provided at the bottom of the tank at a flow rate of 0.2 cm/sec so as to have an average bubble diameter of 2 mm. The surface tension and the contact angle between the droplet of the medium and the water-repellent material were 65 dyne/cm$^2$ and 90°, respectively. The oxygen travel speed in the above aeration was 7 mmol $O_2$/1•hr. In the culture, the cell concentration reached $5.2 \times 10^6$/ml. The concentrations of ammonia and lactic acid produced during the culture were 35 ppm and 1050 ppm, respectively. The dissolved oxygen concentration was maintained at 2 to 5 ppm during the culture. Bubbles on the surface of the medium formed during the culture were completely broken in the debubbling layer 5.

Example 7

The following test was conducted under the same culture tank and culture conditions as those described above, except that a different debubbling layer was used. The debubbling layer 5 used was one prepared by using a stainless steel net having square meshes (size of one mesh: 8 mm) as a skeleton, depositing a polyurethane resin on the stainless steel net so as to have a mesh size of 5 mm, and impregnating the net with polysiloxane ($2 \times 10^3$ cP) in an amount of 5%.

The mixture was cultured for 5 days while introducing sterilized air through a porous glass nozzle 4 provided at the bottom of the tank at a flow rate of 0.2 cm/sec so as to have an average bubble diameter of 2 mm.

The contact angle between the droplet of the medium and the water-repellent material was 85°. The oxygen travel speed in the above aeration was 7 mmol $O_2$/1•hr. In the culture, the cell concentration reached $5.2 \times 10^6$/ml. The concentrations of ammonia and lactic acid produced during the culture were 36 ppm and 1040 ppm, respectively. The dissolved oxygen concentration was maintained at 6 ppm during the culture. Bubbles on the surface of the medium formed during the culture were completely broken on the undersurface of the debubbling layer 5. The cell concentration reached $5.1 \times 10^6$/ml after culture for 5 days.

Comparative Example 6

Culture was conducted in a liquid surface aeration system as opposed to the submerged aeration system of Example 6.

A culture tank having the same shape and volume as those of the culture tank of Example 6 was provided, and the debubbling layer 5 and the submerged aeration nozzle 4 were removed. Instead, an aeration pipe and an exhaust pipe were disposed in the gaseous phase portion opposite to each other and parallel to the surface of the medium. The same cell line was cultured in the same manner to prepare a seed medium. Culture was conducted for 3 days at the same air flow rate by making use of the same volume of the seed medium and the liquid medium and serum of the same lot. The oxygen travel speed was 0.3 mmol $O_2$/1•hr. The dissolved oxygen concentration was 0 to 0.2 ppm in the first day of the culture and 8 ppm after 3 days of initiation of the culture. The cell concentration was $0.9 \times 10^6$/ml. Comparative Example 6 is an example of the prior art, and in the liquid surface aeration system, no sufficient amount of the dissolved oxygen necessary for the proliferation of the cells could be supplied, so that the cell concentration was about 1/6 of that of Example 6.

Comparative Example 7

Culture was conducted in the same manner as that of Example 6, except that the debubbling layer 5 comprised a polyethylene honeycomb layer coated with an olive oil (viscosity: 22 cP). The contact angle relative to the medium was 25°. Bubbles formed through the submerged aeration were brought into contact with the debubbling layer, which exhibited the effect of debubbling for 3 hr. However, thereafter the debubbling layer exhibited no effect, and bubbles overflowed from the exhaust opening 24 provided on the top of the tank over the debubbling layer, which made it impossible to continue the operation. Further, it was observed that the olive oil diffused in the form of an oil film on the surface of the medium.

As is apparent from the above Comparative Example, although the debubbling through direct contact of a low-viscosity water-repellent with the medium is effective for a short period of time, the debubbling effect hardly persists for a long period of time.

Examples 8 to 15

The established animal cells used in Example 6 were cultured by making use of the same liquid medium as that of Example 6. A glass tank having a diameter of 15 cm and a height of 30 cm was used as the culture tank 2. Various debubbling layers 5 shown in the table were provided parallel to the bottom of the tank at a position about 10 cm above the bottom of the tank. Further, a ring sparger having a diameter of 10 cm (a hole diameter of 1 mm; 12 holes) was provided at the bottom of the tank, and aseptic air containing 5% (V/V) carbon dioxide mixed therein was fed at a flow rate shown in Table 1. The temperature was automatically adjusted to 30°C with an external jacket. The culture was conducted for 4 days, and the debubbling effect of the debubbling layer, pressure loss, and the maximum cell concentration attained during the culture period were determined. The results are shown in Table 1. All the debubbling layers exhibited a debubbling effect.

However, an excessively small hole diameter as shown in Example 8 (r = 0.5 mm) brings about an increase in the pressure loss.

Table 1

| Ex. No. | | | 8 | | | | 9 | | | 10 | | | 11 | | |
|---|---|---|---|---|---|---|---|---|---|---|---|---|---|---|---|
| Debubbling layer structure | | | Fig. 21 | | | | Fig. 22 | | | Fig. 23 | | | Fig. 24 | | |
| r (mm) | | | 0.5 | 1 | 2 | 5 | 1 | 2 | 5 | 1 | 2 | 5 | 1 | 2 | 5 |
| h (mm) | | | — | | | | 10 | | | — | | | 10 | | |
| Debubbling effect (O: completely debubbled X: impossible to debubble) | Gas flow rate (cm/sec) | 0.01 | O | | | | O | | | O | | | O | | |
| | | 0.03 | O | | | | O | | | O | | | O | | |
| | | 0.01 | O | | | | O | | | O | | | O | | |
| Pressure loss at 0.1 cm/sec (kg/cm²) | | | 0.1 | 0.04 | <0.01 | | 0.04 | <0.01 | | 0.04 | <0.01 | | 0.04 | <0.01 | |
| Max. cell concentration (cells/ml) | | | $2.7 \times 10^7$ | — | $2.6 \times 10^7$ | $2.5 \times 10^7$ | $2.7 \times 10^7$ | $2.5 \times 10^7$ | $2.7 \times 10^7$ | $2.6 \times 10^7$ | $2.7 \times 10^7$ | $2.5 \times 10^7$ | $2.6 \times 10^7$ | $2.7 \times 10^7$ | $2.6 \times 10^7$ |

EP 0 336 966 B1

Table 2

| Ex. No. | 12 | 13 | 14 | 15 |
|---|---|---|---|---|
| Debubbling layer structure | Fig. 25 | Fig. 26 | Fig. 27 | Fig. 28 |
| r (mm) | 2, 5 | 5, 8 | 3, 5 | 3, 5 |
| h (mm) | 5, 8 | 5, 10 | 5, 10 | 5, 10 |
| Debubbling effect (O: completely debubbled, X: impossible to debubble) — Gas flow rate 0.01 (cm/sec) | O | O | O | O |
| Debubbling effect — Gas flow rate 0.03 (cm/sec) | O | O | O | O |
| Debubbling effect — Gas flow rate 0.01 (cm/sec) | O | O | O | O |
| Pressure loss at 0.1 cm/sec (kg/cm²) | $<0.01$ | $<0.01$ | $<0.01$ | $<0.01$ |
| Max. cell concentration (cells/ml) | $2.6 \times 10^7$ | $2.6 \times 10^7$ | $2.6 \times 10^7$ | $2.7 \times 10^7$ |

Comparative Example 8

Culture was conducted by making use of the same type of culture tank as that of Example 8 under the same condition as that of Example 8, except that only the debubbling layer 5 was different from that of Example 8. The debubbling layer 5 was one prepared by welding stainless steel wires each having a diameter of 1 mm and a length of 5 cm in a cross form on a plane, and the intersection was located at the center portion of the plane cross section of the tank. That is, a debubbling layer provided with an arcuate hole having a diameter of 50 mm and an internal angle of 90° was used. Debubbling could be conducted when the gas flow rate was 0.01 cm/s. However, when the air flow rate was 0.1 cm/sec, bubbles brought about short pass, i.e., were passed through the arcuate hole without being broken, and overflowed from the exhaust opening 24, which made it impossible to continue the operation. The above-described Comparative Example substantiates that the debubbling effect is lowered when the hole diameter of the debubbling layer 5 is excessively large.

Example 16 (not covering the invention)

Hela cells (human uterine cancer cells) were inoculated in a cell concentration of $5 \times 10^4$/ml on a 500-ml glass roller culture bottle containing 100 ml of the same medium as that of Example 6 and cultured for 3 days at 37°C and a speed of rotation of 10 rpm. Air was used as a gaseous phase gas.

The cells adhering to the surface of the culture bottle thus cultured were released to prepare 100 ml of a seed medium having a cell concentration of $1.1 \times 10^5$/ml.

Then, a cylindrical glass culture tank 2 having a diameter of 104 mm, a height of 164 mm, and a volume of 1,500 ml was charged with 100 ml of the above-described seed cell suspension and 705 ml of a liquid medium, and 90 ml of fetal calf serum (900 ml in total), and the mixture was maintained at 37°C. A first debubbling layer comprising stearic acid-coated Teflon® having a lattice interval of 3 mm was provided 5 mm above the surface of the medium, and a lattice of the same type as that described above was provided as a second debubbling layer 3 mm above the first debubbling layer in the perpendicular direction thereof. The mixture was cultured for 5 days while introducing sterilized air through a porous glass nozzle 4 provided at the bottom of the tank at a flow rate of 0.3 cm/sec so as to have an average bubble diameter of 1.5 mm. The surface tension and the contact angle between the droplet of the medium and the water-repellent material were 5 dyne/cm$^2$ and 85°, respectively. The oxygen travel speed in the above aeration was 7 mmol $O_2$/1•hr.

The cell concentration reached $4.2 \times 10^6$/ml. The concentrations of ammonia and lactic acid produced during the culture were 29 ppm and 900 ppm, respectively. The dissolved oxygen concentration was maintained at 2 to 5 ppm during the culture. Bubbles on the surface of the medium formed during the culture were completely broken in the debubbling layer 5 provided in a two-stage form.

Example 17

150 ml of a liquid medium having the following composition was placed in 20 Sakaguchi flasks each having a capacity of 500 ml:

| | |
|---|---|
| soybean extract (a liquid obtained by adding 900 ml of water to 100 g of soybean, conducting heat extraction at 120°C for 30 min, and filtering the extract) | 987 g |
| peptone | 10 g |
| potassium phosphate | 2 g |
| magnesium sulfate hexahydrate | 0.5 g |
| calcium chloride | 0.5 g |
| sodium chloride | 0.5 g |
| pH 7.0 | |

Cells of <u>Bacillus</u> <u>subtilis</u> which had been subjected to aerobic stationary culture in a tests tube containing an agar gel slant medium was inoculated on each flask in a cell concentration of $1.1 \times 10^6$/ml and subjected to aerobic shaking culture at an amplitude of 7 cm and 120 strokes. The medium thus obtained was used as a preliminary medium for the following main culture.

A culture tank 2 having an inner diameter of 16 cm and a height of 25 cm was charged with 2.5 ℓ of a liquid medium having the above-described composition and subjected to steam sterilization at 120°C for 20 min. The culture tank had, at a position 1 cm above the surface of the medium, a debubbling layer 5 made of a porous stainless steel plate having a diameter of 16 cm and a thickness of 0.5 mm (a hole diameter of 3 mm; an opening ratio of 50%) and coated with dimethylpolysiloxane (viscosity: $1 \times 10^5$ cP) and, at the bottom thereof, a ring sparger (a hole diameter of 1 mm; 12 holes) and a paddle agitating blade.

The culture was conducted under the following conditions:

gas flow rate: 0.2 m/sec (the oxygen travel speed was 10 mmol $O_2/1 \cdot hr$)

temperature: 350°C

pH: automatically adjusted to 7.0

(neutralizer: 4N NaOH)

rate of agitation: 50 rpm

Although the amount of bubbles formed in an early stage of the culture was small, a large amount of bubbles were formed after 8 hr of initiation of the culture. However, the bubbles were easily broken in the debubbling layer 5, and no bubbles overflowed on top of the culture tank.

The state of proliferation during culture is shown in Table 3.

Table 3

| culture time (hr) | cell concentration (cells/ml) |
|---|---|
| 0 | $1.1 \times 10^6$ |
| 10 | $1.6 \times 10^7$ |
| 15 | $2.5 \times 10^7$ |
| 20 | $2.4 \times 10^7$ |

Comparative Example 8

Culture was started under the same condition as that of Example 9, except that the debubbling layer 5 of the culture tank used in Example 17 was removed.

The feed of the air was started at a flow rate of 0.2 cm/sec. Eight hours after the initiation of feed of air, a large amount of bubbles were formed on the surface of the medium, and several seconds later, bubbles overflowed into the exhaust opening 24 of the culture tank. Since this made it impossible to continue the operation, the culture was stopped at this stage.

Comparative Example 9

Culture was strated under the same condition as that of Example 8, except that the debubbling layer 5 of the culture tank used in Example 17 was removed and a debubbling blade having a width of 1 cm was mounted and rotated at 500 rpm.

However, when the feed of the air was started at a flow rate of 0.2 cm/sec, bubbles were formed on the surface of the medium, and several seconds later, bubbles overflowed into the exhaust opening 24 of the culture tank. Since this made it impossible to continue the operation, the culture was stopped at this stage.

Comparative Example 10

Culture was conducted in the same manner as that of Comparative Example 9, except that the air was fed at a flow rate of 0.01 cm/sec, i.e., the upper limit of the flow rate with respect to the debubbling effect of the rotary debubbling blade. The oxygen travel speed at the above-described flow rate of air was 0.02 mmol $O_2/1 \cdot hr$. The dissolved oxygen concentration during culture was measured by the electrode method and found to be 0 ppm.

The state of proliferation during culture is shown in Table 4.

23

Table 4

| culture time (hr) | cell concentration (cells/ml) |
|---|---|
| 0 | $1.1 \times 10^6$ |
| 10 | $2.0 \times 10^6$ |
| 15 | $4.2 \times 10^6$ |
| 20 | $4.2 \times 10^6$ |

As is apparent from the above results, in the liquid surface aeration and mechanical debubbling by the rotary blade each known to the art, bubbles remaining on the surface of the medium inhibit the dissolution of oxygen in the medium, so that the proliferation of the cells is as low as 1/6 of that of Example 17.

Example 18

1 ℓ of the following liquid medium was added to a 2 ℓ glass culture tank (7 cm in diameter x 52 cm in height), and Chlorella pyrenoidza was inoculated thereto so as to have a cell concentration of $1 \times 10^5$/ml. A fluorescent lamp was installed on the side surface of the culture tank so that the luminous intensity on the inside wall of the tank was $5 \times 10^3$ luxes. The temperature was adjusted to 30°C with an external jacket. A polybutadiene net (square holes; 5 x 5 mm) impregnated with dimethylpolysilane (viscosity: $7 \times 10^5$ cP) was provided 1 cm above the surface of the medium contained in the culture tank. Further, a ring sparger (a diameter of 5 cm; a hole diameter of 1 mm; 8 holes) was provided at the bottom of the tank. Air containing 5% (V/V) carbon dioxide mixed therein was fed at a flow rate of 0.1 cm/sec.

| | |
|---|---|
| yeast extract | 1 g |
| malt extract | 1 g |
| liver extract | 1 g |
| peptone | 0.3 g |
| glucose | 0.3 g |
| dextrin | 2 g |
| potassium nitrate | 2 g |
| magnesium nitrate hexahydrate | 0.5 g |
| potassium phosphate | 0.01 g |
| ferrous nitrate | 0.005 g |
| pH 7.0 | |

Since the amount of bubbles formed on the surface of the medium in an early stage of the culture was small, bubbles could be sufficiently broken by the debubbling layer. After 5 hr of initiation of the culture, a large amount of bubbles were formed on the surface of the medium. However, these bubbles could be effectively broken by the debubbling layer 5. Therefore, no bubbles overflowed over the debubbling layer throughout the culture.

The state of proliferation during culture is shown in Table 5.

24

Table 5

| culture time (hr) | cell concentration (cells/ml) |
|---|---|
| 0 | $1.1 \times 10^4$ |
| 1 | $2.8 \times 10^4$ |
| 2 | $1.8 \times 10^6$ |
| 4 | $7.0 \times 10^6$ |
| 6 | $7.5 \times 10^6$ |
| 8 | $6.9 \times 10^6$ |

Comparative Example 11

Culture was conducted under the same condition as that of Example 18, except that the debubbling layer of the culture tank was removed.

The feed of air was started at a flow rate of 0.1 cm/sec. Bubbles formed on the surface of the medium spontaneously broke for a culture period of 12 hr. However, 13 hr after the initiation of culture, the gaseous phase portion of the culture tank was filled with bubbles, and the bubbles overflowed into the exhaust opening 24, which caused an aseptic filter connected to the exhaust opening to be clogged with cells. Since this made it impossible to continue the operation, the culture was stopped at this stage.

[Industrial Applicability]

According to the present invention, it is possible to withdraw a cell-free culture filtrate without causing damage to cells and clogging of the membrane by a shearing force of a fluid through filtration of a medium with a hydrophobic membrane. By virtue of this, the medium contained in the culture tank can be efficiently conducted, and the cells can be stably cultured in a high concentration for a long period of time, which renders the invention of the instant application very useful for the production of insulin or the like.

**Claims**

1. A method for culturing cells comprising:
   providing a culture broth in which living cells are suspended in a culture tank,
   placing a water-repellant debubbling structure at a position above a surface of said culture broth, and
   performing culturing of the cells in the broth,
   wherein a debubbling structure is used which comprises gas permeable pores and a surface formed of a liquid organosilicone polymer water-repellant material having a viscosity of at least $1 \times 10^4$ cP.

2. A cell culture apparatus for use in performing the method of claim 1, which comprises:
   a culture tank (2) for holding a culture broth in which living cells are suspended; and
   a water-repellant debubbling structure (5) provided in said culture tank (2) at a level above a surface of said culture broth,
   wherein said water-repellant debubbling structure has gas-permeable pores and has at least a surface thereof formed of a liquid organosilicone polymer water-repellant material having a viscosity of at least $1 \times 10^4$ cP.

3. The apparatus of claim 2, wherein the surface of said debubbling layer (5) has water repellancy such that the contact angle thereof relative to a droplet of said medium is at least 30°.

4. The apparatus of claim 2 or 3, wherein said debubbling layer (5) has a porosity of at least 50%.

25

**5.** The apparatus of claim 2, 3 or 4, wherein said debubbling layer (5) has pores with a size of 2 to 50 mm.

**Patentansprüche**

**1.** Verfahren zur Züchtung von Zellen umfassend:

Bereitstellen einer Kulturlösung, in der lebende Zellen in einem Kulturbehälter suspendiert sind,

Anordnen einer wasserabweisenden Entschäumungsstruktur in einer Position oberhalb der Kulturlösungsoberfläche und

Durchführen der Züchtung der Zellen in der Lösung,

wobei eine Entschäumungsstruktur verwendet wird, die gasdurchlässige Poren sowie eine Oberfläche aufweist, die aus einem flüssigen, organosilikon-polymeren, wasserabweisenden Material mit einer Viskosität von zumindest $1 \times 10^4$ cP gebildet ist.

**2.** Zellenzüchtungsvorrichtung zur Verwendung bei der Durchführung des Verfahrens nach Anspruch 1, umfassend:

einen Kulturbehälter (2) zur Aufnahme einer Kulturlösung, in der lebende Zellen suspendiert sind; und

eine wasserabweisende Entschäumungsstruktur (5), die in dem Kulturbehälter (2) in einer Höhe oberhalb der Kulturlösungsoberfläche angeordnet ist,

wobei die wasserabweisende Entschäumungsstruktur gasdurchlässige Poren aufweist und zumindest eine Oberfläche aus einem flüssigen, organosilikon-polymeren, wasserabweisenden Material mit einer Viskosität von mindestens $1 . 10^4$ cP aufweist.

**3.** Vorrichtung nach Anspruch 2, wobei die Oberfläche der Entschäumungsschicht (5) derartig wasserabweisend ist, daß der Kontaktwinkel relativ zu einem Tropfen des Mediums mindestens 30° beträgt.

**4.** Vorrichtung nach Anspruch 2 oder 3, wobei die Entschäumungsschicht (5) eine Porosität von mindestens 50% aufweist.

**5.** Vorrichtung nach Anspruch 2, 3 oder 4, wobei die Entschäumungsschicht (5) Poren mit einer Größe von 2 bis 50 mm aufweist.

**Revendications**

**1.** Procédé de culture cellulaire comprenant les étapes consistant à :

obtenir un milieu de culture disposé dans une cuve de culture et liquide dans lequel des cellules vivantes sont mises en suspension,

mettre en place une structure hydrofuge de débullage au-dessus de la surface dudit milieu de culture liquide, et

réaliser la culture cellulaire dans ce milieu de culture liquide,

dans lequel on utilise une structure de débullage qui comporte des pores perméables aux gaz et une surface formée d'une substance hydrofuge à base de polymère liquide organo-siliconé dont la viscosité est d'au moins $1 \times 10^4$ cP.

**2.** Dispositif de culture cellulaire pour la mise en oeuvre du procédé de la revendication 1, comprenant :

une cuve de culture (2) contenant le milieu de culture liquide dans lequel les cellules vivantes sont en suspension ; et

une structure hydrofuge de débullage (5) placée dans ladite cuve de culture (2), à un niveau situé au-dessus de la surface dudit milieu de culture liquide,

dans lequel ladite structure hydrofuge de débullage comporte des pores perméables au gaz et a au moins une surface formée d'un polymère liquide hydrofuge organosiliconé possédant une viscosité d'au moins $1 \times 10^4$ cP.

**3.** Dispositif de la revendication 2, dans lequel le caractère hydrofuge de la surface de ladite structure de débullage (5) est tel que son angle de contact avec une gouttelette dudit milieu est d'au moins 30°.

4. Dispositif de la revendication 2 ou 3, dans lequel ladite couche de débullage (5) présente une porosité d'au moins 50 %.

5. Dispositif de la revendication 2, 3 ou 4 dans lequel ladite couche de débullage (5) possède des pores d'une taille de 2 à 50 mm.

FIG. 1

# FIG. 2

# FIG. 3

# FIG. 4

# FIG. 5

# FIG. 6

FIG. 7

FIG. 8A

FIG. 8B

FIG. 9A

FIG. 9B

FIG. 10

FIG. 11

FIG. 12

FIG. 13

## FIG. 14

# FIG. 15

# FIG. 16

FIG. 17

FIG. 18

## FIG. 19

## FIG. 20

FIG. 21

FIG. 22

FIG. 23

FIG. 24

FIG. 25

FIG. 26

FIG. 27

FIG. 28